# EUROPEAN PATENT SPECIFICATION

(11) **EP 3 484 516 B1**
(45) Date of publication and mention of the grant of the patent: **02.09.2026**
(21) Application number: 17828588.8
(22) Date of filing: 14.07.2017
(51) Int. Cl.: A61K 39/395, C07K 16/28, C12N 15/09, A61P 35/00

(54) **MULTIPLE BI-SPECIFIC BINDING DOMAIN CONSTRUCTS WITH DIFFERENT EPITOPE BINDING TO TREAT CANCER**
MEHRERE BISPEZIFISCHE BINDUNGSDOMÄNENKONSTRUKTE MIT UNTERSCHIEDLICHER EPITOPBINDUNG ZUR BEHANDLUNG VON KREBS
CONSTRUCTIONS MULTIPLES DE DOMAINES DE LIAISON BI-SPÉCIFIQUES AYANT UNE LIAISON À L'ÉPITOPE DIFFÉRENTE POUR TRAITER LE CANCER

(30) Priority: 14.07.2016 US 201662362397 P; 31.03.2017 US 201762480230 P
(43) Date of publication of application: 22.05.2019
(73) Proprietor: Fred Hutchinson Cancer Center, Seattle, WA 98109 (US)
(72) Inventor: MEHLIN, Christopher, Seattle Washington 98109 (US); CORRENTI, Colin, Seattle Washington 98109 (US); OLSON, James, Seattle Washington 98109 (US); WALTER, Roland B., Seattle Washington 98109 (US); BANDARANAYAKE, Ashok, Seattle Washington 98109 (US); LASZLO, George S., Seattle Washington 98109 (US)
(74) Representative: Forresters IP LLP
(86) International application number: PCT/US2017/042264
(87) International publication number: WO 2018/014001

(56) References cited:
- WO-A1-2012/055961
- WO-A1-2014/167022
- WO-A1-2015/184203
- WO-A2-2014/144722
- WO-A2-2016/086189
- US-A1- 2011 165 161
- US-A1- 2013 251 642
- US-A1- 2014 242 081
- US-A1- 2015 306 141
- SURYADEVARA CARTER M ET AL: "Are BiTEs the 'missing link' in cancer therapy?", ONCOIMMUNOLOGY,, vol. 4, no. 6, 1 June 2015 (2015-06-01), pages e1008339 - 1, XP008178923, ISSN: 2162-402X, [retrieved on 20150430], DOI: 10.1080/2162402X.2015.1008339
- GOEBELER MARIA-ELISABETH ET AL: "Blinatumomab: a CD19/CD3 bispecific T cell engager (BiTE) with unique anti-tumor efficacy.", LEUKEMIA & LYMPHOMA MAY 2016, vol. 57, no. 5, May 2016 (2016-05-01), pages 1021 - 1032, XP002796823, ISSN: 1029-2403
- ZHU MIN ET AL: "Blinatumomab, a Bispecific T-cell Engager (BiTE( )) for CD-19 Targeted Cancer Immunotherapy: Clinical Pharmacology and Its Implications.", CLINICAL PHARMACOKINETICS 10 2016, vol. 55, no. 10, 21 May 2016 (2016-05-21), pages 1271 - 1288, XP002796824, ISSN: 1179-1926
- ZUGMAIER GERHARD ET AL: "Clinical overview of anti-CD19 BiTE and ex vivo data from anti-CD33 BiTE as examples for retargeting T cells in hematologic malignancies", MOLECULAR IMMUNOLOGY, PERGAMON, GB, vol. 67, no. 2, 13 April 2015 (2015-04-13), pages 58 - 66, XP029246900, ISSN: 0161-5890, DOI: 10.1016/J.MOLIMM.2015.02.033
- LASZLO G S ET AL: "T-cell ligands modulate the cytolytic activity of the CD33/CD3 BiTE antibody construct, AMG 330", BLOOD CANCER JOURNAL,, vol. 5, 1 August 2015 (2015-08-01), XP002793582, DOI: 10.1038/BCJ.2015.68
- KIMBERLY H. HARRINGTON ET AL: "The Broad Anti-AML Activity of the CD33/CD3 BiTE Antibody Construct, AMG 330, Is Impacted by Disease Stage and Risk", PLOS ONE, vol. 10, no. 8, 25 August 2015 (2015-08-25), pages e0135945, XP055370197, DOI: 10.1371/journal.pone.0135945
- KRUPKA C ET AL: "Blockade of the PD-1/PD-L1 axis augments lysis of AML cells by the CD33/CD3 BiTE antibody construct AMG 330: reversing a T-cell-induced immune escape mechanism", LEUKEMIA, SPRINGER NATURE PUBLISHING AG, vol. 30, no. 2, 4 August 2015 (2015-08-04), pages 484 - 491, XP002763683, ISSN: 1476-5551
- GURUNADH R. CHICHILI ET AL: "A CD3xCD123 bispecific DART for redirecting host T cells to myelogenous leukemia: Preclinical activity and safety in nonhuman primates", SCIENCE TRANSLATIONAL MEDICINE, vol. 7, no. 289, 27 May 2015 (2015-05-27), US, pages 289ra82 - 289ra82, XP055653881, ISSN: 1946-6234, DOI: 10.1126/scitranslmed.aaa5693
- AL-HUSSAINI MUNEERA ET AL: "Targeting CD123 in acute myeloid leukemia using a T-cell-directed dual-affinity retargeting platform.", BLOOD 07 JAN 2016, vol. 127, no. 1, 7 January 2016 (2016-01-07), pages 122 - 131, XP002796825, ISSN: 1528-0020
- KLINGER MATTHIAS ET AL: "Harnessing T cells to fight cancer with BiTE antibody constructs--past developments and future directions.", IMMUNOLOGICAL REVIEWS MAR 2016, vol. 270, no. 1, March 2016 (2016-03-01), pages 193 - 208, XP002796826, ISSN: 1600-065X
- SATYEN HARISH GOHIL ET AL: "An ROR1 bi-specific T-cell engager provides effective targeting and cytotoxicity against a range of solid tumors", ONCOIMMUNOLOGY, 17 May 2017 (2017-05-17), pages e1326437, XP055500395, DOI: 10.1080/2162402X.2017.1326437
- JUDITH FEUCHT ET AL: "T-cell responses against CD19+ pediatric acute lymphoblastic leukemia mediated by bispecific T-cell engager (BiTE) are regulated contrarily by PD-L1 and CD80/CD86 on leukemic blasts", ONCOTARGET, vol. 7, 30 September 2016 (2016-09-30), XP055355709, DOI: 10.18632/oncotarget.12357
- GOHIL ET AL: "Preclinical development of novel humanised ROR1 targeting chimeric antigen receptor T cells and bispecific T-cell engagers", LANCET, THE, 1 January 2017 (2017-01-01), London, pages S40 - S40, XP055500396, Retrieved from the Internet <URL:https://www.thelancet.com/action/showPdf?pii=S0140-6736(17)30436-1> DOI: 10.1016/S0140-6736(17)30436-1

## Description

### CROSS-REFERENCE TO RELATED APPLICATIONS

This application claims priority to 62/362,397 filed on July 14, 2016 and 62/480,230 filed on March 31, 2017.

### FIELD OF THE DISCLOSURE

The present disclosure provides multiple bi-specific binding domain constructs (BS-BDC) to treat cancer or stimulate immune response in a subject in need thereof. Each BS-BDC within a group targets a cancer antigen epitope and an immune cell activating epitope that is different from the cancer antigen epitope and immune cell activating epitope targeted by another BS-BDC within the group. The different cancer antigen epitopes can be on the same cancer antigen.

### BACKGROUND OF THE DISCLOSURE

Despite advances in cancer treatments, mortality associated with the disease remains too high. For example, despite improvements in outcome for many pediatric patients, cancer remains the leading cause of death past infancy among children in the United States. Thus, the need for effective new therapies for cancers, including childhood cancers is unquestioned.

Targeting cancer cells with antibodies raised high expectations as a potent means of eliminating tumor cells with limited non-specific toxicities. For many patients, however, use of single antibodies has not been effective.

Bispecific T-cell engaging antibodies bind both a cancer antigen on tumor cells and a T cell activating epitope, with the goal of bringing T cells to cancer cells to destroy the cancer cells. See, for example, US 2008/0145362. Current bispecific T-cell engaging antibody therapeutics include pairs of monospecific, antibody-derived binding domains. One member of the pair targets a cancer antigen epitope and the other member of the pair targets a T cell activating epitope. Some have explored use of such antibodies in combinations that target two different T cell activating epitopes (e.g., CD3 and CD28). Unfortunately, this approach similarly has not achieved the hoped for therapeutic efficacy. WO2014/167022 discloses bispecific antibodies which bind to ROR1 and CD3. Thus, there remains a dire need in the art for more effective cancer therapies, especially for those with more refractory or difficult to treat cancer types.

Progress has been made in genetically engineering T cells of the immune system to target and kill unwanted cell types, such as cancer cells. For example, T cells have been genetically engineered to express molecules having extracellular components that bind particular target antigens and intracellular components that direct actions of the T cell when the extracellular component has bound the target antigen. As an example, the extracellular component can be designed to bind target antigens found on cancer cells and, when bound, the intracellular component directs the T cell to destroy the bound cancer cell. Examples of such molecules include genetically engineered T cell receptors (TCR) and chimeric antigen receptors (CAR).

While TCR and/or CAR-modified T cells provide a major advantage in that they can create immune memory against cancer cells that can attack recurrent or progressive cancer cells as they emerge over time, this immune memory can lead to autoimmune toxicities when they recognize targets on normal tissue as abnormal or foreign.

### SUMMARY OF THE DISCLOSURE

The present invention is defined by the claims. The present disclosure provides multiple bi-specific binding domain constructs (BS-BDC) to treat cancer or stimulating an immune response in a subject in need thereof. Each BS-BDC within a group binds a cancer antigen epitope that is non-overlapping and non-repetitive with a cancer antigen epitope targeted by another BS-BDC within the group, and an immune cell activating epitope that is that is non-overlapping and non-repetitive with an immune cell activating epitope targeted by another BS-BDC within the group, wherein two of the non-overlapping and non-repetitive cancer antigen epitopes are located on ROR1 and one of the non-overlapping and non-repetitive immune cell activating epitopes is located on CD3 and one of the non-overlapping and non-repetitive immune cell activating epitopes is located on CD28. The different cancer antigen epitopes are on the same cancer antigen, and are non-overlapping different cancer antigen epitopes on the same cancer antigen. This advance provides several benefits. First, because BS-BDC within a group bind different cancer antigen epitopes, there is less competition for binding and reduced steric hindrance. Second, by binding different immune cell activating epitopes, immune cell co-stimulation signals are achieved. Because the binding domains recognizing the immune cell activating epitopes (e.g., a T-cell receptor and a co-stimulatory receptor), are located on different BS-BDC within a group, the group will induce T-cell activation only in the presence of cancer cells. This approach provides a versatile platform that can be utilized to target a large variety of cancers.

Use of the described groups of BS-BDC targeting at least two cancer antigen epitopes and at least two immune cell activating epitopes provided unexpected synergistic effects on T cell mediated killing of cancer cells. Moreover, use of the described groups of BS-BDC unexpectedly overcame cancer cell resistance to single bispecific T-cell engaging antibody constructs.

Additional benefits of the disclosed approach over many currently available therapies include that the disclosed BS-BDC can be provided as an "off-the-shelf" therapy that can be administered universally to patients with a particular cancer without the need for personalized genetic therapies, such as CAR-modified T-cell therapies that can lead to autoimmune toxicities. Further, individual patient responses to the therapy can be monitored and dosages correspondingly adjusted to avoid adverse treatment effects such as cytokine storms. The therapy also activates T cells specifically at the site of a cancer, as opposed to infusing activated T cells into patients, relying on tumor homing of the infused pre-activated cells.

The disclosed BS-BDC can also be used in combinations that track the course of an individual patient's disease over time. For example, CD28 provides an immune cell activating epitope expressed on T cells. Following on-going T cell activation, however (as in the tumor microenvironment), T cells can down-regulate expression of CD28 over time resulting in reduced opportunities for T cell activation through this epitope. Accordingly, while a treatment may beneficially begin with a BS-BDC that binds CD28, over time this BS-BDC may be replaced with one that binds an epitope that reverses or blocks the activation an inhibitory T cell epitope (e.g., 4-1BB (CD 137), PD-1, TIM-3, LAG3, VISTA). Many such beneficial evolving combinations of BS-BDC groups are described herein.

For all of the foregoing reasons, the described groups of BS-BDC provide an important and significant advance in the on-going fight against cancer.

### BRIEF DESCRIPTION OF THE FIGURES

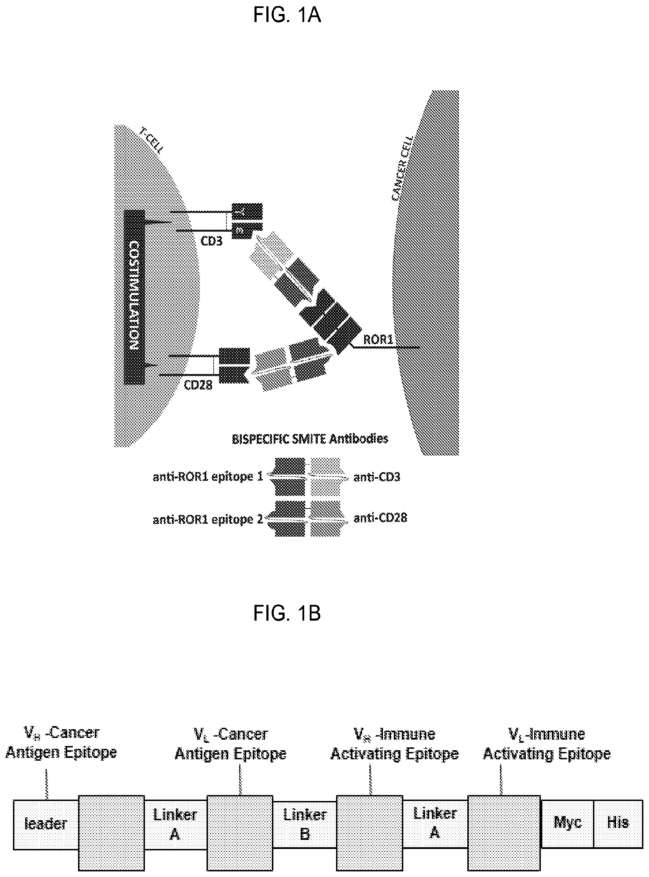

FIGs. 1A and 1B. (1A) Depiction of Simultaneous Multiple Interaction T-cell Engaging BS-BDC engaged with a T-cell and a cancer cell. In this depicted embodiment, one BS-BDC in a group binds an epitope on ROR1 and CD3. A second BS-BDC in the group binds a different epitope on ROR1 and CD28 on the same T cell. (1B) An exemplary BS-BDC format.

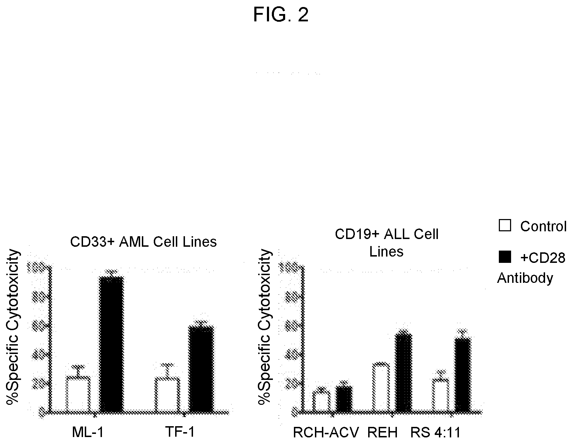

FIG. 2. Cytotoxicity of cancer cell/T-cell BS-BDC upon T-cell co-stimulation.
FIGs. 3A and 3B. T-cell co-activation with CD28-directed BS-BDC is strictly dependent on presence of target antigen-positive cancer cells.
FIGs. 4A-4D. T-cell co-activation with CD28-directed BS-BDC augments ROR1/CD3 antibody-induced cytotoxicity.
FIG. 5. T-cell co-activation with CD28-directed BS-BDC targeting a second cancer cell antigen augments anti-cancer activity of a therapeutic bispecific T-cell engaging antibody.
FIGs. 6A-6C. PD-L1/CD28 antibody can overcome PD-L1-mediated resistance to bispecific antibodies.

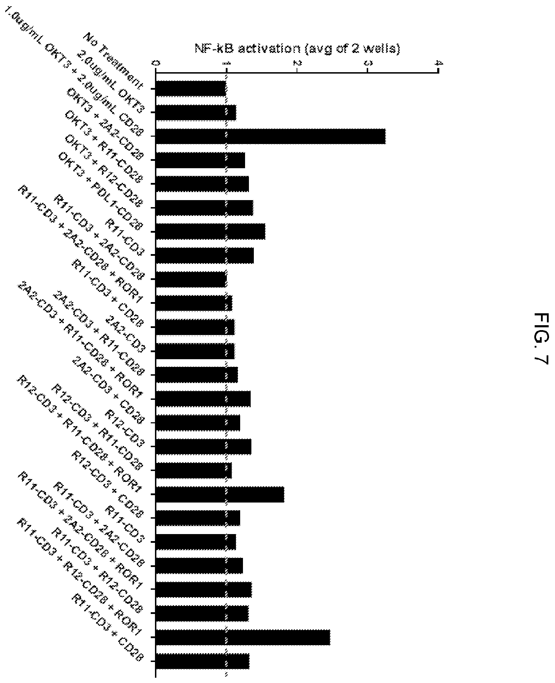

FIG. 7. R11 and 2A2 bind different but overlapping ROR1 epitopes while R12 binds a different and non-overlapping ROR1 epitope, as measured using an NFkB reporter Jurkat line.
FIG. 8. Supporting sequences.

### DETAILED DESCRIPTION

Despite advances in cancer treatments, mortality associated with the disease remains too high. For example, despite improvements in outcome for many pediatric patients, cancer remains the leading cause of death past infancy among children in the United States. Thus, the need for effective new therapies for cancers, including childhood cancers is unquestioned.

Targeting cancer cells with antibodies raised high expectations as a potent means of eliminating tumor cells with limited non-specific toxicities. For many patients, however, use of single antibodies has not been effective.

Bispecific T-cell Engaging antibodies bind both a cancer antigen on tumor cells and a T cell activating epitope, with the goal of bringing T cells to cancer cells to destroy the cancer cells. See, for example, US 2008/0145362. Current bispecific T-cell engaging antibody therapeutics include pairs of monospecific, antibody-derived binding domains. One member of the pair targets a cancer antigen epitope and the other member of the pair targets a T cell activating epitope. Some have explored use of such antibodies in combinations that target two different T cell activating epitopes (e.g., CD3 and CD28). Unfortunately, this approach similarly has not achieved the hoped for therapeutic efficacy. Thus, there remains a dire need in the art for more effective cancer therapies, especially for those with more refractory or difficult to treat cancer types.

The present disclosure provides multiple bi-specific binding domain constructs (BS-BDC; e.g., bi-specific antibodies) to treat cancer or stimulate an immune response in a subject in need thereof. Each BS-BDC in a group binds a cancer antigen epitope that is non-overlapping and non-repetitive with a cancer antigen epitope targeted by another BS-BDC within the group, and an immune cell activating epitope that is non-overlapping and non-repetitive with an immune cell activating epitope targeted by another BS-BDC in the group, wherein two of the non-overlapping and non-repetitive cancer antigen epitopes are located on ROR1 and one of the non-overlapping and non-repetitive immune cell activating epitopes is located on CD3 and one of the non-overlapping and non-repetitive immune cell activating epitopes is located on CD28. The different cancer antigen epitopes are on the same cancer antigen, and are non-repetitive different cancer antigen epitopes on the same cancer antigen. This advance provides several benefits. First, because each BS-BDC in a group binds a different cancer antigen epitope, there is less competition for binding and reduced steric hindrance. Second, by binding different immune cell activating epitopes, co-stimulation signaling is achieved. Because binding domains recognizing the T-cell receptor and the co-stimulatory receptor are located on different BS-BDC, the BS-BDC group will induce T-cell activation only in the presence of cancer cells. This approach provides a versatile platform that can be utilized to target a large variety of cancers.

Use of the described groups of BS-BDC targeting at least two cancer antigen epitopes and at least two immune cell activating epitopes provided unexpected synergistic effects on T cell mediated killing of cancer cells. Moreover, use of the described groups of BS-BDC unexpectedly overcame cancer cell resistance to single bispecific T-cell engaging antibody constructs.

Additional benefits of the disclosed approach over many currently available therapies include that the disclosed BS-BDC can be provided as an "off-the-shelf" therapy that can be administered universally to patients with a particular cancer without the need for personalized genetic therapies, such as CAR-modified T-cell therapies. Further, individual patient responses to the therapy can be monitored and dosages correspondingly adjusted to avoid adverse treatment effects such as cytokine storms. The therapy also activates T cells specifically at the site of a cancer, as opposed to infusing activated T cells into patients, relying on tumor homing of the infused pre-activated cells.

The disclosed BS-BDC can also be used in combinations that track the course of an individual patient's disease over time. In particular embodiments, the administered BS-BDC can change over the course of a treatment regimen based on immune system status (e.g., stage of activation), stage of response to treatment, and/or change in cancer antigens expressed by cancer cells. The change between BS-BDC can occur at least 1 hour following administration of a first BS-BDC or up to several days, weeks, or months following administration of a first BS-BDC. In particular embodiments, changes in administered in BS-BDC are based on on-going subject monitoring, by for example, feedback from subject samples (e.g., blood tests). In particular embodiments, changes in administered in BS-BDC can be pre-programmed based on predictable changes in immune status and/or cancer cycle or expected responses to treatment. In particular embodiments, changes in administered in BS-BDC can be pre-programmed and automatically made based on, for example, use of a programmable pump. Changes in administered BS-BDC can occur acutely or can shift gradually.

In particular embodiments, a patient can be monitored for changes in immune activation, and the BS-BDCs administered can be changed to BS-BDCs that target a different immune activating epitope. As one example, CD28 provides an immune cell activating epitope expressed on T cells. Following on-going T cell activation, however (as in the tumor microenvironment), T cells can down-regulate expression of CD28 over time resulting in reduced opportunities for T cell activation through this epitope. Accordingly, while a treatment may beneficially begin with a BS-BDC that binds CD28, over time this BS-BDC may be replaced with one that binds an epitope that reverses or blocks the activation an inhibitory T cell epitope (e.g., 4-1BB (CD 137), PD-1, TIM-3, LAG3, VISTA). Many such beneficial evolving combinations of BS-BDC groups are described herein.

In particular embodiments, a patient can be monitored for changes in cancer antigen expression, and the BS-BDCs administered can be switched to BS-BDCs that target a different cancer antigen. Cancer antigen expression often changes during the course of cancer. As one example, Her-2, the molecular target of the cancer drug trastuzumab, can become down-regulated during treatment, leading to treatment resistance (Shi et al. Breast Cancer Research 2014 16: R33). In particular aspects of the disclosure, a patient being treated with BS-BDCs that target Her-2 can be monitored for Her-2 downregulation, and if their cancer loses or reduces Her-2 expression, they can be treated with BS-BDCs that target a different cancer antigen. EGFR is another example of a cancer antigen that becomes down-regulated during the course of treatment.

During the course of treatment, administered BS-BDC groups can evolve to change targeted cancer antigens, targeted immune activating epitopes, or both. Changes can reflect addition of a targeted cancer antigen and/or immune cell activating epitope; removal of a targeted cancer antigen and/or immune cell activating epitope; and/or replacement of a targeted cancer antigen and/or immune cell activating epitope.

For all of the foregoing reasons, the described groups of BS-BDC provide an important and significant advance in the on-going fight against cancer.

As indicated, "different from" means that the targeted epitopes are distinct from one another in sequence and/or structure. In particular embodiments, in addition to being different, targeted epitopes are also non-overlapping. "Non-overlapping" means that the binding of one BS-BDC in a group to an epitope is not decreased to a statistically-significant degree in a competitive binding assay by the presence of at least one other BS-BDC in the group. Non-overlapping epitopes may be epitopes on different molecules (e.g., ROR1 and CD33; CD3 and CD28) or may be non-overlapping epitopes located on the same molecule (e.g., non-overlapping ROR1 epitopes; non-overlapping CD3 epitopes). Non-repetitive different epitopes on the same antigen exclude epitopes that are physically distinct in space from one another yet repetitive in sequence to each other. For example, MUC1 has a repetitive sequence, and the repeats within the sequence are not non-repetitive and different, as defined herein.

"Co-stimulation" of T cells means that a more robust T cell response is observed in the presence of members of a BS-BDC group than in the presence of one member of the BS-BDC group alone.

In particular embodiments, the BS-BDC groups disclosed herein can be referred to as SMITE groups. SMITE stands for "Simultaneous Multiple Interaction T-Cell Engaging" binding domain constructs (e.g., antibodies, scFv). This terminology reflects the fact that the disclosed BS-BDC group will engage two different immune cell activating epitopes when bound to two different cancer antigen epitopes. Engagement of the immune cell activating epitopes will overlap in time, causing robust T cell activation at the site of cancer cells.

Groups of BS-BDC can include two, three, or four BS-BDC. If a group includes two BS-BDC (a pair), each member of the pair will bind a different cancer antigen epitope and a different immune cell activating epitope. If a group includes three BS-BDC, each member of the group can bind a different cancer antigen epitope and a different immune cell activating epitope (targeting three cancer antigen epitopes and three immune cell activating epitopes), or two members of a three member group may target the same cancer antigen epitope and two members of the three member group may target the same immune cell activating epitope. The same principle applies to four member groups. That is, if a group includes four BS-BDC, each member of the group can bind a different cancer antigen epitope and a different immune cell activating epitope (targeting four cancer antigen epitopes and four immune cell activating epitopes). Alternatively, a subset of members of the group can target a common cancer antigen epitope and/or immune cell activating epitope. No matter the number of members, each grouping will target at least two different cancer antigen epitopes and, in particular embodiments, at least two different immune cell activating epitopes. In particular embodiments, each grouping will target at least two different cancer antigen epitopes and a common immune cell activating epitope (e.g., CD3 or CD28).

The disclosed groups of BS-BDC provide a versatile platform that can be utilized to target a large variety of cancers, such as adrenal cancers, bladder cancers, blood cancers, bone cancers, brain cancers, breast cancers, carcinoma, cervical cancers, colon cancers, colorectal cancers, corpus uterine cancers, ear, nose and throat (ENT) cancers, endometrial cancers, esophageal cancers, gastrointestinal cancers, head and neck cancers, Hodgkin's disease, intestinal cancers, kidney cancers, larynx cancers, leukemias, liver cancers, lymph node cancers, lymphomas, lung cancers, melanomas, mesothelioma, myelomas, nasopharynx cancers, neuroblastomas, non-Hodgkin's lymphoma, oral cancers, ovarian cancers, pancreatic cancers, penile cancers, pharynx cancers, prostate cancers, rectal cancers, sarcoma, seminomas, skin cancers, stomach cancers, teratomas, testicular cancers, thyroid cancers, uterine cancers, vaginal cancers, vascular tumors, and metastases thereof.

Aspects of the disclosure are now described in more detail.

BS-BDC Formats. BS-BDC formats include a protein with a first binding domain that binds a cancer antigen epitope and a second binding domain that binds an immune cell activating epitope. Exemplary bispecific antibody formats are described in, e.g., WO2009/080251, WO2009/080252, WO2009/080253, WO2009/080254, WO2010/112193, WO2010/115589, WO2010/136172, WO2010/145792, and WO2010/145793.

Different binding domains can be derived from multiple sources such as antibodies, fibronectin, affibodies, natural ligands (e.g., CD80 and CD86 for CD28), etc. In particular embodiments, binding domains can be derived from whole antibodies or binding fragments of an antibody, e.g., Fv, Fab, Fab', F(ab')₂, Fc, and single chain Fv fragments (scFvs) or any biologically effective fragments of an immunoglobulin that bind specifically to a cancer antigen epitope or immune cell activating epitope (e.g., T cell receptor). Antibodies or antigen binding fragments include all or a portion of polyclonal antibodies, monoclonal antibodies, human antibodies, humanized antibodies, synthetic antibodies, chimeric antibodies, bispecific antibodies, mini bodies, and linear antibodies.

BS-BDC including binding domains from human origin or humanized antibodies have lowered immunogenicity in humans and have a lower number of non-immunogenic epitopes compared to non-human antibodies. Binding domains will generally be selected to have reduced antigenicity in human subjects. Binding domains can particularly include any peptide that specifically binds a selected cancer antigen epitope or immune cell activating epitope. Sources of binding domains include antibody variable regions from various species (which can be in the form of antibodies, sFvs, scFvs, Fabs, scFv-based grababody, or soluble VH domain or domain antibodies). These antibodies can form antigen-binding regions using only a heavy chain variable region, *i.e.,* these functional antibodies are homodimers of heavy chains only (referred to as "heavy chain antibodies") (Jespers et al., Nat. Biotechnol. 22:1161, 2004; Cortez-Retamozo et al., Cancer Res. 64:2853, 2004; Baral et al., Nature Med. 12:580, 2006; and Barthelemy et al., J. Biol. Chem. 283:3639, 2008).

Phage display libraries of partially or fully synthetic antibodies are available and can be screened for an antibody or fragment thereof that can bind a selected epitope. For example, binding domains may be identified by screening a Fab phage library for Fab fragments that specifically bind to a target of interest (*see* Hoet et al., Nat. Biotechnol. 23:344, 2005). Phage display libraries of human antibodies are also available. Additionally, traditional strategies for hybridoma development using a target of interest as an immunogen in convenient systems (*e.g*., mice, HuMAb mouse^{®}, TC mouse^{™}, KM-mouse^{®}, llamas, chicken, rats, hamsters, rabbits, *etc.*) can be used to develop binding domains. In particular embodiments, binding domains specifically bind to selected epitopes expressed by targeted cancer cells and/or T cells and do not cross react with nonspecific components or unrelated targets. Once identified, the amino acid sequence or polynucleotide sequence coding for the CDR within a binding domain can be isolated and/or determined.

An alternative source of binding domains includes sequences that encode random peptide libraries or sequences that encode an engineered diversity of amino acids in loop regions of alternative non-antibody scaffolds, such as scTCR (*see, e.g.,* Lake et al., Int. Immunol.11:745, 1999; Maynard et al., J. Immunol. Methods 306:51, 2005; U.S. Patent No. 8,361,794), mAb² or Fcab^{™} (*see, e.g.,* PCT Patent Application Publication Nos. WO 2007/098934; WO 2006/072620), affibodies, avimers, fynomers, cytotoxic T-lymphocyte associated protein-4 (Weidle et al., Cancer Gen. Proteo. 10:155, 2013), and the like (Nord et al., Protein Eng. 8:601, 1995; Nord et al., Nat. Biotechnol. 15:772, 1997; Nord et al., Euro. J. Biochem. 268:4269, 2001; Binz et al., Nat. Biotechnol. 23:1257, 2005; Boersma and Plückthun, Curr. Opin. Biotechnol. 22:849, 2011).

In particular embodiments, an antibody fragment is used as one or more binding domains in a BS-BDC. An "antibody fragment" denotes a portion of a complete or full length antibody that retains the ability to bind to an epitope. Examples of antibody fragments include Fv, scFv, Fab, Fab', Fab'-SH, F(ab')₂; diabodies; and linear antibodies.

A single chain variable fragment (scFv) is a fusion protein of the variable regions of the heavy and light chains of immunoglobulins connected with a short linker peptide. Fv fragments include the VL and VH domains of a single arm of an antibody. Although the two domains of the Fv fragment, VL and VH, are coded by separate genes, they can be joined, using, for example, recombinant methods, by a synthetic linker that enables them to be made as a single protein chain in which the VL and VH regions pair to form monovalent molecules (single chain Fv (scFv)). For additional information regarding Fv and scFv, see e.g., Bird, et al., Science 242

(1988) 423-426; Huston, et al., Proc. Natl. Acad. Sci. USA 85 (1988) 5879-5883; Plueckthun, in The Pharmacology of Monoclonal Antibodies, vol. 113, Rosenburg and Moore (eds.), Springer-Verlag, New York), (1994) 269-315; WO1993/16185; US Patent 5,571,894; and US Patent 5,587,458.

A Fab fragment is a monovalent antibody fragment including VL, VH, CL and CH1 domains. A F(ab')₂ fragment is a bivalent fragment including two Fab fragments linked by a disulfide bridge at the hinge region. For discussion of Fab and F(ab')₂ fragments having increased *in vivo* half-life, see U.S. Patent 5,869,046. Diabodies include two epitope-binding sites that may be bivalent. See, for example, EP 0404097; WO1993/01161; and Holliger, et al., Proc. Natl. Acad. Sci. USA 90 (1993) 6444-6448. Dual affinity retargeting antibodies (DART^{™}; based on the diabody format but featuring a C-terminal disulfide bridge for additional stabilization (Moore et al., Blood 117, 4542-51 (2011))) can also be used. Antibody fragments can also include isolated CDRs. For a review of antibody fragments, see Hudson, et al., Nat. Med. 9 (2003) 129-134.

Antibody fragments can be made by various techniques, including proteolytic digestion of an intact antibody as well as production by recombinant host-cells (e.g. human suspension cell lines, E. coli or phage), as described herein. Antibody fragments can be screened for their binding properties in the same manner as intact antibodies.

In particular embodiments, BS-BDC can also include a natural receptor or ligand for an epitope as a binding domain. For example, if a target for binding includes PD-L1, the binding domains can include PD-1 (including, e.g., a PD-1/antiCD3 fusion). One example of a receptor fusion for binding is Enbrel^{®} (Amgen). Natural receptors or ligands can also be modified to enhance binding. For example, betalacept is a modified version of abatacept. In particular embodiments, the BS-BDC can include a natural receptor or ligand that induces phagocytosis. Calreticulin (UniProt ID No. P27797) is a protein that is localized to the endoplasmic reticulum of healthy cells, but in dying cells it translocates to the cell surface and induces phagocytosis by immune cells such as macrophages. In particular embodiments, the binding domains can include calreticulin or a portion of calreticulin that is capable of inducing phagocytosis.

Binding can also be enhanced through increasing avidity which arises from multimerizaiton of the binding domain. Any screening method known in the art can be used to identify increased avidity to an antigen epitope.

In particular embodiments, the BS-BDC format can be based on blinatumomab with binding domains selected for particularly targeted cancer antigens and immune cell activating epitopes. In particular embodiments, the BS-BDC format can be based on AMG330 with binding domains selected for particularly targeted cancer antigens and immune cell activating epitopes.

In particular embodiments, the BS-BDC formats can include a single chain antibody attached to the C-terminus of a light chain (see, e.g., Oncoimmunology. 2017; 6(3): e1267891). This format can be useful because the presence of the Fc region can help preserve the protein half-life. The presence of the Fc region can also be useful because Fc interacts with several receptors and can contribute to the immune response. Antibody-scFv fusions can also be useful because the antibody portion binds to its epitope in a dimeric fashion, which enhances avidity and the scFv portion binds its epitope in a monomeric fashion, which can be useful, for example, for binding T-cell epitopes and only allowing multimerization in the presence of a target (e.g., cancer cell). These embodiments can be "tri-specific".

For a review of additional BS-BDC formats that can be used, see Brinkmann & Kontermann, mAbs, 2017. 9:2, 182-212, DOI: 10.1080/19420862.2016.1268307.

An "epitope" includes any determinant capable of being bound by an antigen-binding protein, such as an antibody or a T-cell receptor. An epitope is a region of an antigen that is bound by an antigen binding protein that targets that antigen, and when that antigen is a protein, includes specific residues that directly contact the antigen binding protein. In particular embodiments, an "epitope" denotes the binding site on a protein target bound by a corresponding binding domain. The binding domain either binds to a linear epitope, (e.g., an epitope including a stretch of 5 to 12 consecutive amino acids), or the binding domain binds to a three-dimensional structure formed by the spatial arrangement of several short stretches of the protein target. Three-dimensional epitopes recognized by a binding domain, e.g. by the epitope recognition site or paratope of an antibody or antibody fragment, can be thought of as three-dimensional surface features of an epitope molecule. These features fit precisely (in)to the corresponding binding site of the binding domain and thereby binding between the binding domain and its target protein is facilitated. In particular embodiments, an epitope can be considered to have two levels: (i) the "covered patch" which can be thought of as the shadow an antibody or binding domain would cast; and (ii) the individual participating side chains and backbone residues. Binding is then due to the aggregate of ionic interactions, hydrogen bonds, and hydrophobic interactions.

"Bind" means that the binding domain associates with its target epitope with a dissociation constant (1(D) of 10⁻⁸ M or less, in particular embodiments of from 10⁻⁵ M to 10⁻¹³ M, in particular embodiments of from 10⁻⁵ M to 10⁻¹⁰ M, in particular embodiments of from 10⁻⁵ M to 10⁻⁷ M, in particular embodiments of from 10⁻⁸ M to 10⁻¹³ M, or in particular embodiments of from 10⁻⁹ M to 10⁻¹³ M. The term can be further used to indicate that the binding domain does not bind to other biomolecules present, (e.g., it binds to other biomolecules with a dissociation constant (KD) of 10⁻⁴ M or more, in particular embodiments of from 10⁻⁴ M to 1 M). A targeted epitope is one that will be bound by its corresponding BS-BDC binding domain under relevant *in vitro* conditions and in *in vivo* conditions as described herein. In particular embodiments, relevant *in vitro* conditions for binding can include a buffered salt solution approximating physiological pH (7.4) at room temperature or 37°C.

Targeted Cancer Antigen Epitopes. Cancer cell antigens are expressed by cancer cells. One of the significant features of the current disclosure is that the cancer antigen need not be preferentially expressed by cancer cells. This is because meaningful SMITE-induced immune cell activation occurs only in the presence of cancer cells. As one example, PD-L1 is expressed by cancer cells and non-cancer cells.

In particular embodiments, cancer cell antigens are preferentially expressed by cancer cells. "Preferentially expressed" means that a cancer cell antigen is found at higher levels on cancer cells as compared to other cell types. In some instances, a cancer antigen is only expressed by the targeted cancer cell type. In other instances, the cancer antigen is expressed on the targeted cancer cell type at least 25%, 35%, 45%, 55%, 65%, 75%, 85%, 95%, 96%, 97%, 98%, 99%, or 100% more than on non-targeted cells.

The following table provides examples of particular cancers and cancer antigens that can be targeted with BS-BDC.

| Targeted Cancer | Cancer Antigens |
|---|---|
| Leukemia/Lymphoma | CD19, CD20, CD22, ROR1, CD33, WT-1, CD123 |
| Multiple Myeloma | B-cell maturation antigen (BCMA) |
| Prostate Cancer | PSMA, WT1, Prostate Stem Cell antigen (PSCA), SV40 T |
| Breast Cancer | HER2, ERBB2, ROR1 |
| Stem Cell Cancer | CD133 |
| Ovarian Cancer | L1-CAM, extracellular domain of MUC16 (MUC-CD), folate binding protein (folate receptor), Lewis Y, ROR1, mesothelin, WT-1 |
| Mesothelioma | mesothelin |
| Renal Cell Carcinoma | carboxy-anhydrase-IX (CAIX); |
| Melanoma | GD2 |
| Pancreatic Cancer | mesothelin, CEA, CD24, ROR1 |
| Lung Cancer | ROR1 |

In more particular examples, cancer cell antigens include:

| Cancer Antigen | Sequence |
|---|---|
| PSMA | KSSNEATNITPKHNMKAFLDELKAENIKKFLYNFTQIPHLAGTEQNFQLAKQIQ SQWKEFGLDSVELAHYDVLLSYPNKTHPNYISIINEDGNEIFNTSLFEPPPPGY ENVSDIVPPFSAFSPQGMPEGDLVYVNYARTEDFFKLERDMKINCSGKIVIAR YGKVFRGNKVKNAQLAGAKGVILYSDPADYFAPGVKSYPDGWNLPGGGVQ |
| | RGNILNLNGAGDPLTPGYPANEYAYRRGIAEAVGLPSIPVHPIGYYDAQKLLE KMGGSAPPDSSWRGSLKVPYNVGPGFTGNFSTQKVKMHIHSTNEVTRIYNV IGTLRGAVEPDRYVILGGHRDSWVFGGIDPQSGAAVVHEIVRSFGTLKKEGW RPRRTILFASWDAEEFGLLGSTEWAEENSRLLQERGVAYINADSSIEGNYTL RVDCTPLMYSLVHNLTKELKSPDEGFEGKSLYESWTKKSPSPEFSGMPRISK LGSGNDFEVFFQRLGIASGRARYTKNWETNKFSGYPLYHSVYETYELVEKFY DPMFKYHLTVAQVRGGMVFELANSIVLPFDCRDYAVVLRKYADKIYSISMKH PQEMKTYSVSFDSLFSAVKNFTEIASKFSERLQDFDKSNPIVLRMMNDQLMF LERAFIDPLGLPDRPFYRHVIYAPSSHNKYAGESFPGIYDALFDIESKVDPSKA WGEVKRQIYVAAFTVQAAAETLSEVA (SEQ ID NO: 1) |
| PSCA | MKAVLLALLMAGLALQPGTALLCYSCKAQVSNEDCLQVENCTQLGEQCWTA RIRAVGLLTVISKGCSLNCVDDSQDYYVGKKNITCCDTDLCNASGAHALQPA AAILALLPALGLLLWGPGQL (SEQ ID NO: 2) |
| Mesothelin |  (SEQ ID NO: 3) |
| CD19 |  (SEQ ID NO: 4) |
| CD20 | MTTPRNSVNGTFPAEPMKGPIAMQSGPKPLFRRMSSLVGPTQSFFMRESKT LGAVQIMNGLFHIALGGLLMIPAGIYAPICVTVWYPLWGGIMYIISGSLLAATEK NSRKCLVKGKMIMNSLSLFAAISGMILSIMDILNIKISHFLKMESLNFIRAHTPYI NIYNCEPANPSEKNSPSTQYCYSIQSLFLGILSVMLIFAFFQELVIAGIVENEWK RTCSRPKSNIVLLSAEEKKEQTIEIKEEVVGLTETSSQPKNEEDIEIIPIQEEEEE ETETNFPEPPQDQESSPIENDSSP (SEQ ID NO: 5) |
| CD33 (full length) | MPLLLLLPLLWAGALAMDPNFWLQVQESVTVQEGLCVLVPCTFFHPIPYYDK NSPVHGYWFREGAIISRDSPVATNKLDQEVQEETQGRFRLLGDPSRNNCSL SIVDARRRDNGSYFFRMERGSTKYSYKSPQLSVHVTDLTHRPKILIPGTLEPG HSKNLTCSVSWACEQGTPPIFSWLSAAPTSLGPRTTHSSVLIITPRPQDHGT NLTCQVKFAGAGVTTERTIQLNVTYVPQNPTTGIFPGDGSGKQETRAGVVHG |
| | AIGGAGVTALLALCLCLIFFIVKTHRRKAARTAVGRNDTHPTTGSASPKHQKK SKLHGPTETSSCSGAAPTVEMDEELHYASLNFHGMNPSKDTSTEYSEVRTQ (SEQ ID NO: 6) |
| CD33 (DeltaE2 variant) | MPLLLLLPLLWADLTHRPKILIPGTLEPGHSKNLTCSVSWACEQGTPPIFSWL SAAPTSLGPRTTHSSVLIITPRPQDHGTNLTCQVKFAGAGVTTERTIQLNVTY VPQNPTTGIFPGDGSGKQETRAGVVHGAIGGAGVTALLALCLCLIFFIVKTHR RKAARTAVGRNDTHPTTGSASPKHQKKSKLHGPTETSSCSGAAPTVEMDEE LHYASLNFHGMNPSKDTSTEYSEVRTQ (SEQ ID NO: 7) |
| CD33 (with C-terminal truncation) | MPLLLLLPLLWAGALAMDPNFWLQVQESVTVQEGLCVLVPCTFFHPIPYYDK NSPVHGYWFREGAIISRDSPVATNKLDQEVQEETQGRFRLLGDPSRNNCSL SIVDARRRDNGSYFFRMERGSTKYSYKSPQLSVHVTDLTHRPKILIPGTLEPG HSKNLTCSVSWACEQGTPPIFSWLSAAPTSLGPRTTHSSVLIITPRPQDHGT NLTCQVKFAGAGVTTERTIQLNVTYVPQNPTTGIFPGDGSGKQETRAGVVHG AIGGAGVTALLALCLCLIFFIVKTHRRKAARTAVGRNDTHPTTGSASPVR (SEQ ID NO: 8) |
| ROR1 | MHRPRRRGTRPPLLALLAALLLAARGAAAQETELSVSAELVPTSSWNISSEL NKDSYLTLDEPMNNITTSLGQTAELHCKVSGNPPPTIRWFKNDAPVVQEPRR LSFRSTIYGSRLRIRNLDTTDTGYFQCVATNGKEVVSSTGVLFVKFGPPPTAS PGYSDEYEEDGFCQPYRGIACARFIGNRTVYMESLHMQGEIENQITAAFTMI GTSSHLSDKCSQFAIPSLCHYAFPYCDETSSVPKPRDLCRDECEILENVLCQT EYIFARSNPMILMRLKLPNCEDLPQPESPEAANCIRIGIPMADPINKNHKCYNS TGVDYRGTVSVTKSGRQCQPWNSQYPHTHTFTALRFPELNGGHSYCRNPG NQKEAPWCFTLDENFKSDLCDIPACDSKDSKEKNKMEILYILVPSVAIPLAIAL LFFFICVCRNNQKSSSAPVQRQPKHVRGQNVEMSMLNAYKPKSKAKELPLS AVRFMEELGECAFGKIYKGHLYLPGMDHAQLVAIKTLKDYNNPQQWTEFQQ EASLMAELHHPNIVCLLGAVTQEQPVCMLFEYINQGDLHEFLIMRSPHSDVG CSSDEDGTVKSSLDHGDFLHIAIQIAAGMEYLSSHFFVHKDLAARNILIGEQLH VKISDLGLSREIYSADYYRVQSKSLLPIRWMPPEAIMYGKFSSDSDIWSFGVV LWEIFSFGLQPYYGFSNQEVIEMVRKRQLLPCSEDCPPRMYSLMTECWNEIP SRRPRFKDIHVRLRSWEGLSSHTSSTTPSGGNATTQTTSLSASPVSNLSNPR YPNYMFPSQGITPQGQIAGFIGPPIPQNQRFIPINGYPIPPGYAAFPAAHYQPT GPPRVIQHCPPPKSRSPSSASGSTSTGHVTSLPSSGSNQEANIPLLPHMSIP NHPGGMGITVFGNKSQKPYKIDSKQASLLGDANIHGHTESMISAEL (SEQ ID NO: 9) |
| WT1 | IEGRHMRRVPGVAPTLVRSASETSEKRPFMCAYPGCNKRYFKLSHLQMHSR KHTGEKPYQCDFKDCERRFFRSDQLKRHQRRHTGVKPFQCKTCQRKFSRS DHLKTHTRTHTGEKPFSCRWPSCQKKFARSDELVRHHNMHQRNMTKLQLA L (SEQ ID NO: 10) |
| CD123 | MVLLWLTLLLIALPCLLQTKEDPNPPITNLRMKAKAQQLTWDLNRNVTDIECV KDADYSMPAVNNSYCQFGAISLCEVTNYTVRVANPPFSTWILFPENSGKPW AGAENLTCWIHDVDFLSCSWAVGPGAPADVQYDLYLNVANRRQQYECLHY KTDAQGTRIGCRFDDISRLSSGSQSSHILVRGRSAAFGIPCTDKFVVFSQIEIL TPPNMTAKCNKTHSFMHWKMRSHFNRKFRYELQIQKRMQPVITEQVRDRTS FQLLNPGTYTVQIRARERVYEFLSAWSTPQRFECDQEEGANTRAWRTSLLIA LGTLLALVCVFVICRRYLVMQRLFPRIPHMKDPIGDSFQNDKLVVWEAGKAG LEECLVTEVQVVQKT (SEQ ID NO: 11) |

As will be understood by one of ordinary skill in the art, targeted antigens can lack signal peptides, such as the underlined segments of representative CD33 antigens, SEQ ID NOs: 6-8. Further, and as will be understood, "same cancer antigen" allows, does not require that both targeted epitopes be on the same cancer antigen molecule. That is, and for example, when two different epitopes of ROR1 are targeted, one BS-BDC in a group could bind to the first epitope on a first ROR1 molecule and the second BS-BDC in the group could bind the second epitope on a different ROR1 molecule. Similarly, the first and second epitope could be bound by the first and second BS-BDC on the same ROR1 molecule.

In embodiments of the invention, the cancer antigen epitope binding domains present within a BS-BDC group each target a different RORI epitope (e.g., ROR1-A and ROR1-B).

In particular embodiments, the cancer antigen epitope binding domain of at least one BS-BDC in a group is a human or humanized binding domain (e.g., scFv) including a variable light chain including a CDRL1 sequence including ASGFDFSAYYM (SEQ ID NO: 12), a CDRL2 sequence including TIYPSSG (SEQ ID NO: 13), and a CDRL3 sequence including ADRATYFCA (SEQ ID NO: 14). In particular embodiments, the cancer antigen epitope binding domain of at least one BS-BDC in a group is a human or humanized binding domain (e.g., scFv) including a variable heavy chain including a CDRH1 sequence including DTIDWY (SEQ ID NO: 15), a CDRH2 sequence including VQSDGSYTKRPGVPDR (SEQ ID NO: 16), and a CDRH3 sequence including YIGGYVFG (SEQ ID NO: 17).

In particular embodiments, the cancer antigen epitope binding domain of at least one BS-BDC in a group is a human or humanized binding domain (e.g., scFv) including a variable light chain including a CDRL1 sequence including QASQSIDSNLA (SEQ ID NO: 18), a CDRL2 sequence including RASNLAS (SEQ ID NO: 19), and a CDRL3 sequence including LGGVGNVSYRTS (SEQ ID NO: 20). In particular embodiments, the cancer antigen epitope binding domain of at least one BS-BDC in a group is a human or humanized binding domain (e.g., scFv) including a variable heavy chain including a CDRH1 sequence including DYPIS (SEQ ID NO: 21), a CDRH2 sequence including FINSGGSTWYASWVKG (SEQ ID NO: 22), and a CDRH3 sequence including GYSTYYCDFNI (SEQ ID NO: 23). These reflect CDR sequences of the R11 antibody.

In particular embodiments, the cancer antigen epitope binding domain of at least one BS-BDC in a group is a human or humanized binding domain (e.g., scFv) including a variable light chain including a CDRL1 sequence including TLSSAHKTDTID (SEQ ID NO: 24), a CDRL2 sequence including GSYTKRP (SEQ ID NO: 25), and a CDRL3 sequence including GADYIGGYV (SEQ ID NO: 26). In particular embodiments, the cancer antigen epitope binding domain of at least one BS-BDC in a group is a human or humanized binding domain (e.g., scFv) including a variable heavy chain including a CDRH1 sequence including AYYMS (SEQ ID NO: 27), a CDRH2 sequence including TIYPSSGKTYYATWVNG (SEQ ID NO: 28), and a CDRH3 sequence including DSYADDGALFNI (SEQ ID NO: 29). These reflect CDR sequences of the R12 antibody.

In particular embodiments, the cancer antigen epitope binding domain of at least one BS-BDC in a group is a human or humanized binding domain (e.g., scFv) including a variable light chain including a CDRL1 sequence including KASQNVDAAVA (SEQ ID NO: 30), a CDRL2 sequence including SASNRYT (SEQ ID NO: 31), and a CDRL3 sequence including QQYDIYPYT (SEQ ID NO: 32). In particular embodiments, the cancer antigen epitope binding domain of at least one BS-BDC in a group is a human or humanized binding domain (e.g., scFv) including a variable heavy chain including a CDRH1 sequence including DYEMH (SEQ ID NO: 33), a CDRH2 sequence including AIDPETGGTAYNQKFKG (SEQ ID NO: 34), and a CDRH3 sequence including YYDYDSFTY (SEQ ID NO: 35). These reflect CDR sequences of the 2A2 antibody.

In particular embodiments, the cancer antigen epitope binding domain of at least one BS-BDC in a group is a human or humanized binding domain (e.g., scFv) including a variable light chain including a CDRL1 sequence including QASQSIGSYLA (SEQ ID NO: 36), a CDRL2 sequence including YASNLAS (SEQ ID NO: 37), and a CDRL3 sequence including LGSLSNSDNV (SEQ ID NO: 38). In particular embodiments, the cancer antigen epitope binding domain of at least one BS-BDC in a group is a human or humanized binding domain (e.g., scFv) including a variable heavy chain including a CDRH1 sequence including SHWMS (SEQ ID NO: 39), a CDRH2 sequence including IIAASGSTYYANWAKG (SEQ ID NO: 40), and a CDRH3 sequence including DYGDYRLVTFNI (SEQ ID NO: 41). These reflect CDR sequences of the Y31 antibody.

A number of additional antibodies specific for RORI are known to those of skill in the art and can be readily characterized for sequence, epitope binding, and affinity. See, for example, WO2008076868, WO/2008103849, WO201008069, WO2010124188, WO2011079902, WO2011054007, WO2011159847, WO2012076066, WO2012076727, WO 2012045085, and WO2012097313.

In particular aspects of the disclosure, the cancer antigen epitope binding domains present within a BS-BDC group each target a different CD19 epitope. In particular embodiments, cancer antigen epitope binding domain of at least one BS-BDC in a group includes a binding domain (e.g., scFv) that include VH and VL regions specific for CD19. In particular embodiments, the V_{H} and V_{L} regions are human. Exemplary V_{H} and V_{L} regions include the segments of anti-CD19 specific monoclonal antibody FMC63. In particular embodiments, the binding domain (e.g., scFV) is human or humanized and including a variable light chain including a CDRL1 sequence including RASQDISKYLN (SEQ ID NO: 42), a CDRL2 sequence including SRLHSGV (SEQ ID NO: 43), and a CDRL3 sequence including GNTLPYTFG (SEQ ID NO: 44). In particular embodiments, the binding domain (e.g., scFV) is human or humanized and includes a variable heavy chain including a CDRH1 sequence including DYGVS (SEQ ID NO: 45), a CDRH2 sequence including VTWGSETTYYNSALKS (SEQ ID NO: 46), and a CDRH3 sequence including YAMDYWG (SEQ ID NO: 47). Other CD19-targeting antibodies such as SJ25C1 and HD37 are known. (SJ25C1: Bejcek et al. Cancer Res 2005, PMID 7538901; HD37: Pezutto et al. JI 1987, PMID 2437199).

In particular aspects of the disclosure, the cancer antigen epitope binding domains present within a BS-BDC group each target a different PSMA epitope. A number of antibodies specific for PSMA are known to those of skill in the art and can be readily characterized for sequence, epitope binding, and affinity. Binding domains can also include anti-Mesothelin ligands (associated with treating ovarian cancer, pancreatic cancer, and mesothelioma). As will be understood by one of ordinary skill in the art, the different cancer antigen epitope binding domains can bind any number of different epitopes on the cancer antigens disclosed herein (among others). As previously indicated, in particular embodiments, the different epitopes are on the same cancer antigen. In particular embodiments, the different epitopes are on different cancer antigens.

In particular aspects of the disclosure, the cancer antigen epitope binding domains present within a BS-BDC group each target a different CD20 epitope. Rituxan (Rituximab, Genentech) targets CD20 for CD20-positive non-Hodgkin's lymphoma and Arzerra (Ofatumumab, Novartis), targets a different epitope of CD20.

In particular aspects of the disclosure, the cancer antigen epitope binding domain of at least one BS-BDC in a group is a human or humanized binding domain (e.g., scFV) including a variable light chain including a CDRL1 sequence including RASSSVSYIH (SEQ ID NO: 48), a CDRL2 sequence including ATSNLAS (SEQ ID NO: 49), and a CDRL3 sequence including QQWTSNPPT (SEQ ID NO: 50). In particular embodiments, the cancer antigen epitope binding domain of at least one BS-BDC in a group is a human or humanized binding domain (e.g., scFv) including a variable heavy chain including a CDRH1 sequence including SYNMH (SEQ ID NO: 51), a CDRH2 sequence including AIYPGNGDTSYNQKFKG (SEQ ID NO: 52), and a CDRH3 sequence including STYYGGDWYFNV (SEQ ID NO: 53). These reflect CDR sequences of the 2B8 antibody.

In particular aspects of the disclosure, the cancer antigen epitope binding domain of at least one BS-BDC in a group is a human or humanized binding domain (e.g., scFv) including a variable light chain including a CDRL1 sequence including RASQDVNTAVAW (SEQ ID NO: 54), a CDRL2 sequence including YSASFLES (SEQ ID NO: 55), and a CDRL3 sequence including QQHYTTPT (SEQ ID NO: 56). In particular embodiments, the cancer antigen epitope binding domain of at least one BS-BDC in a group is a human or humanized binding domain (e.g., scFv) including a variable heavy chain including a CDRH1 sequence including SGFNTKDTYIHW (SEQ ID NO: 57), a CDRH2 sequence including RIYPTNGYTRYADSVKGR (SEQ ID NO: 58), and a CDRH3 sequence including WGGDGFYAMDV (SEQ ID NO: 59). These reflect CDR sequences of the 4D5 antibody.

In particular embodiments, the cancer antigen epitope binding domains present within a BS-BDC group each target a different CD33 epitope.

In particular embodiments, the BS-BDC binds only full length CD33 (CD33^{FL}), only the splice variant of CD33 that lacks exon 2 (CD33^{ΔE2}); or (iii) CD33 regardless of whether it is CD33^{FL} or CD33^{ΔE2}. Groups of BS-BDC targeting different CD33 isoforms can target a higher percentage of CD33-expressing cells because they can target cells expressing CD33^{FL} and CD33^{ΔE2}. Further, BS-BDC binding CD33^{ΔE2} provides therapeutic targeting for cells that express the CD33^{ΔE2} variant, but that do not express the CD33^{FL} protein.

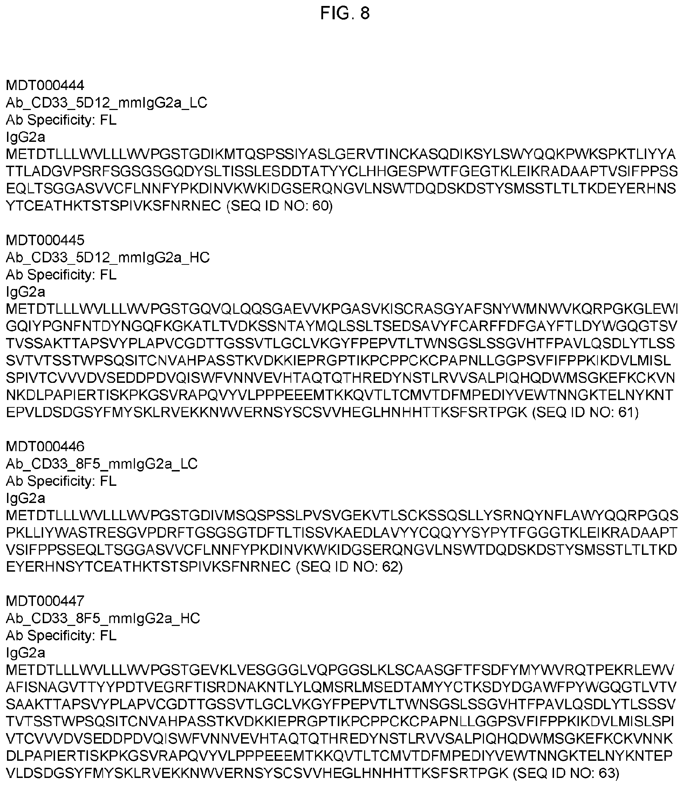

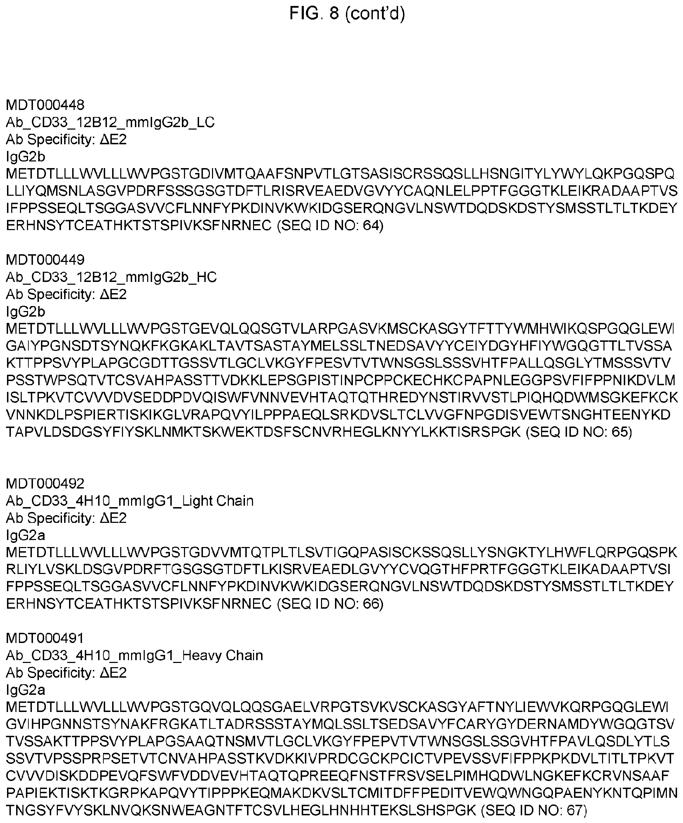

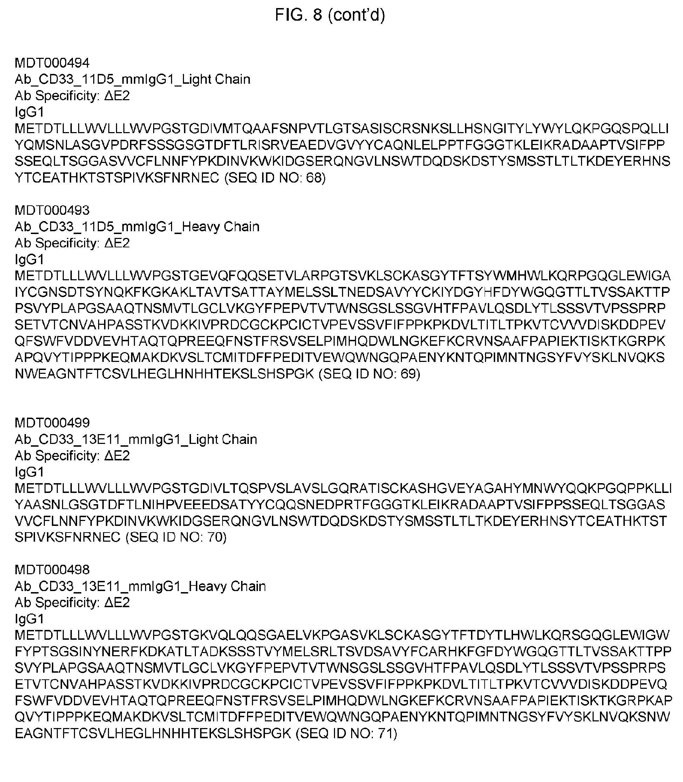

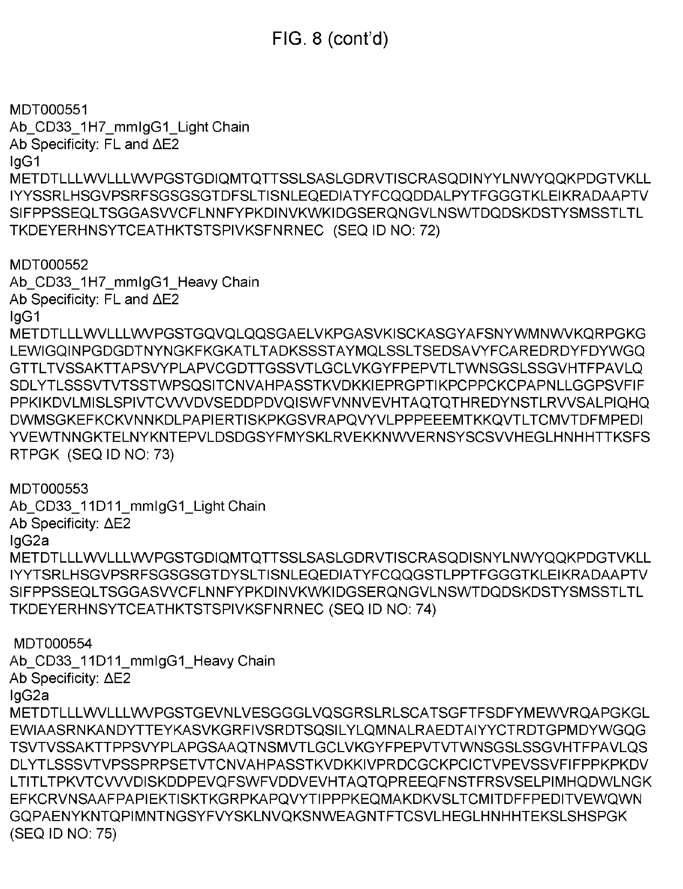

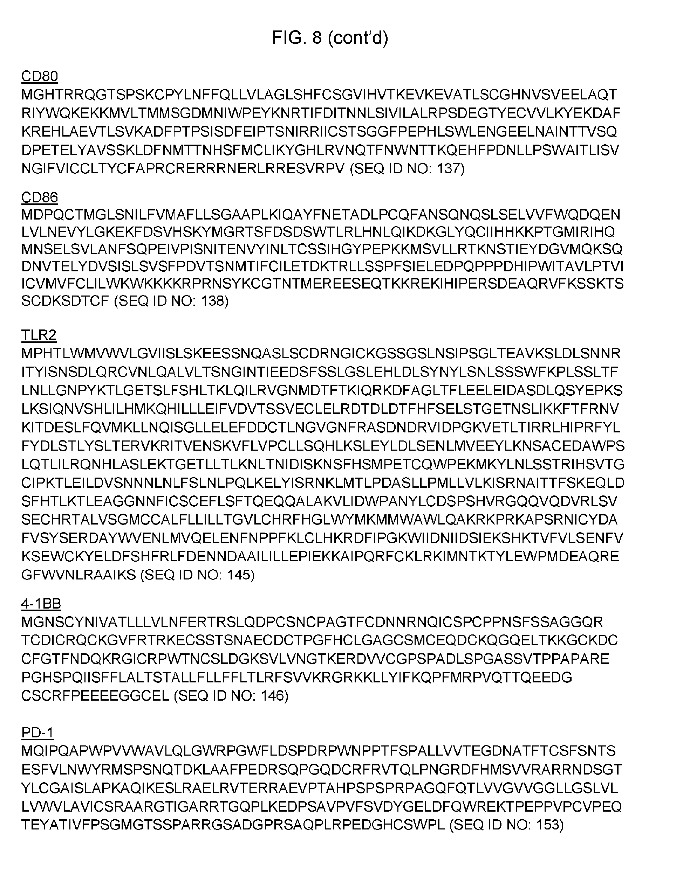

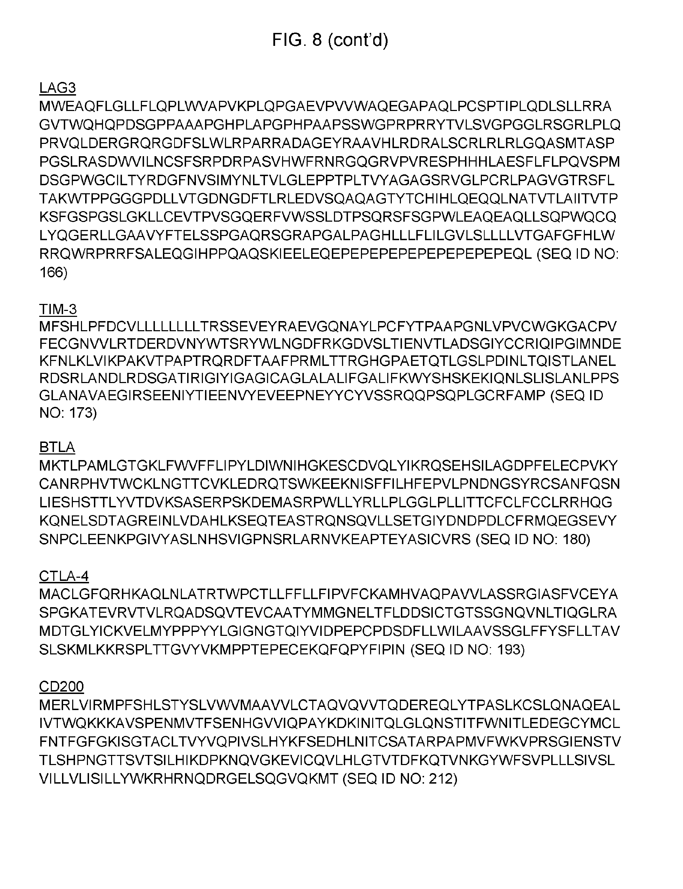

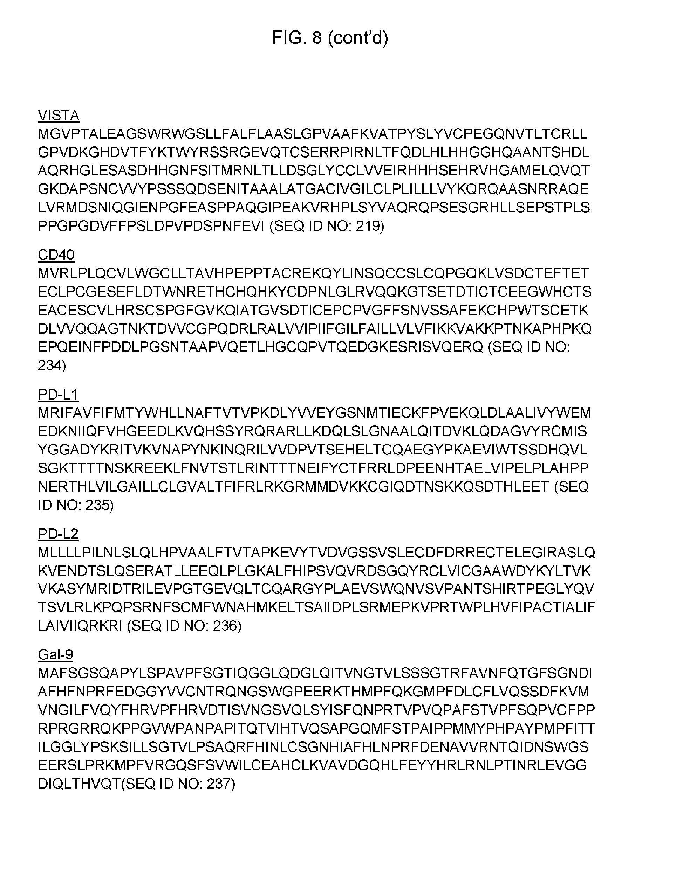

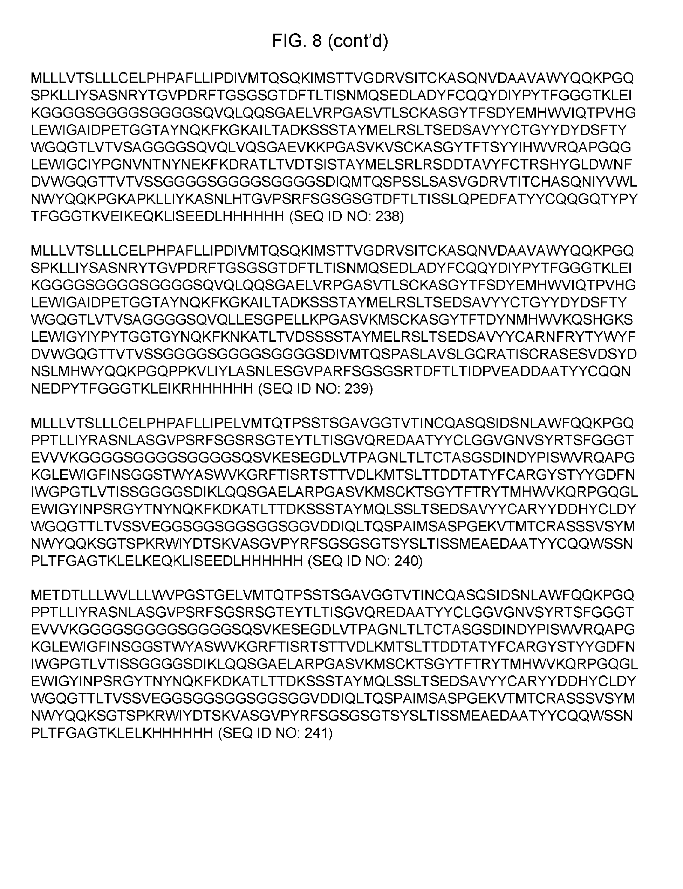

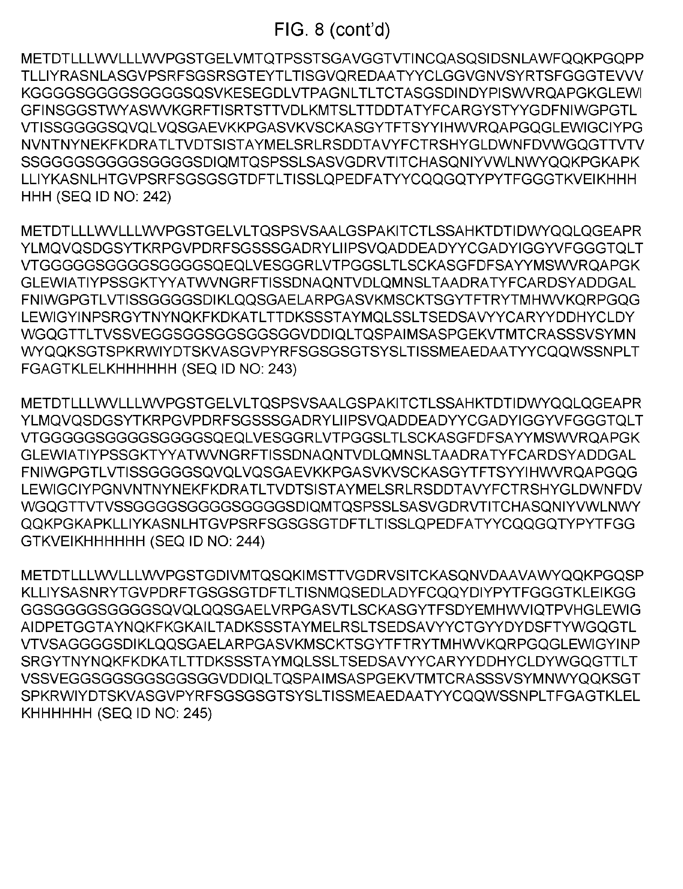

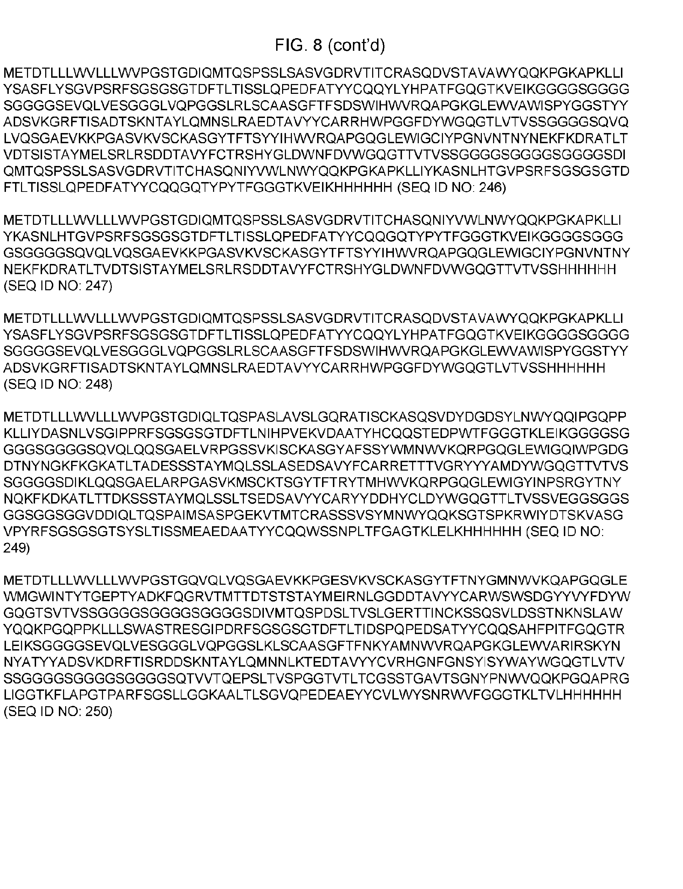

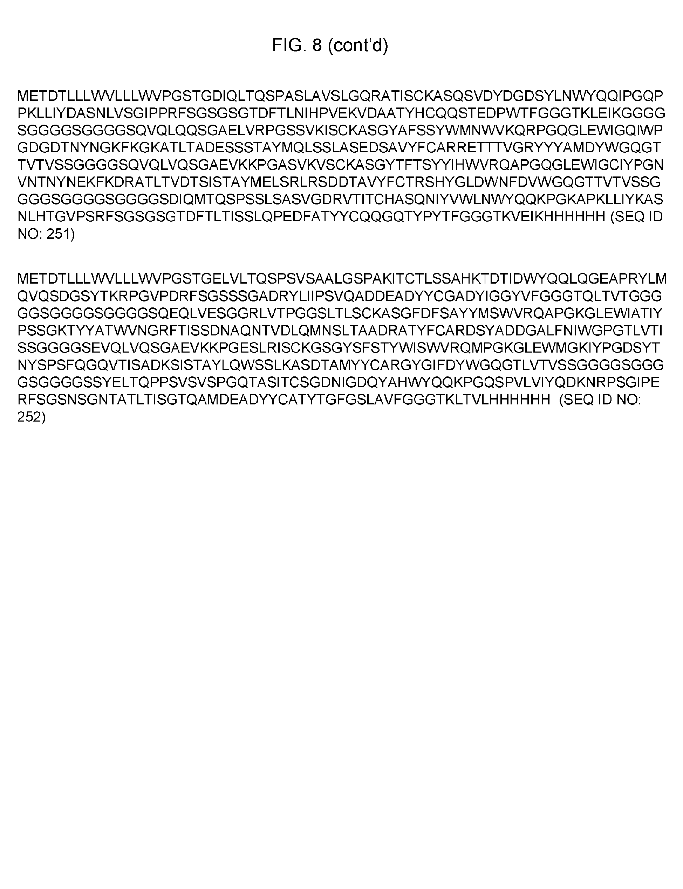

Referring to FIG. 8, the following variable light (V_{L}) and variable heavy (V_{H}) chains are provided for BS-BDC with the following specificities:

| Antibody Name | Specific For | Chain | SEQ ID NO: |
|---|---|---|---|
| 5D12 | CD33^{FL} | V_{L} | 60 |
| | | V_{H} | 61 |
| 8F5 | CD33^{FL} | V_{L} | 62 |
| | | V_{H} | 63 |
| 12B12 | CD33^{ΔE2} | V_{L} | 64 |
| | | V_{H} | 65 |
| 4H10 | CD33^{ΔE2} | V_{L} | 66 |
| | | V_{H} | 67 |
| 11D5 | CD33^{ΔE2} | V_{L} | 68 |
| | | V_{H} | 69 |
| 13E11 | CD33^{ΔE2} | V_{L} | 70 |
| | | V_{H} | 71 |
| 1H7 | CD33^{FL} and CD33^{ΔE2} | V_{L} | 72 |
| | | V_{H} | 73 |
| 11D11 | CD33^{ΔE2} | V_{L} | 74 |
| | | V_{H} | 75 |

Definitive delineation of a CDR and identification of residues including the binding site of an antibody can be accomplished by solving the structure of the antibody and/or solving the structure of the antibody-epitope complex. In particular embodiments, this can be accomplished by methods such as X-ray crystallography.

In particular embodiments, the cancer antigen epitope binding domain of at least one BS-BDC in a group is a human or humanized CD33 binding domain (e.g., scFv) including a variable light chain including a CDRL1 sequence including RASEVDNYGISFMN (SEQ ID NO: 76), a CDRL2 sequence including AASNQGS (SEQ ID NO: 77), and a CDRL3 sequence including QQSKEVPW (SEQ ID NO: 78). In particular embodiments, In particular embodiments, the cancer antigen epitope binding domain of at least one BS-BDC in a group is a human or humanized CD33 binding domain (e.g., scFv) including a variable heavy chain including a CDRH1 sequence including DYNMH (SEQ ID NO: 79), a CDRH2 sequence including YIYPYNGGTGYNQKFKS (SEQ ID NO: 80), and a CDRH3 sequence including GRPAMDY (SEQ ID NO: 81). These reflect CDR sequences of the M195 or the HuM195 antibody.

In particular embodiments, the CD33 binding domain includes a variable light chain including a CDRL1 sequence including (SEQ ID NO: 253), a sequence including (SEQ ID NO: - 254), and a CDRL3 sequence including (SEQ ID NO: 255). In particular embodiments, the CD33 binding domain includes a variable heavy chain including a CDRH1 sequence including (SEQ ID NO: 256), a CDRH2 sequence including (SEQ ID NO: 257), and a CDRH3 sequence including (SEQ ID NO: 258). These reflect the CDR sequences of the 1H7 antibody.

In particular aspects of the disclosure, the cancer antigen epitope binding domains present within a BS-BDC group each target a different PD-L1 epitope. In particular embodiments, the PD-L1 binding domain includes a variable light chain including a CDRL1 sequence including RASQDVSTAVA (SEQ ID NO: 267), a CDRL2 sequence including SASFLYS (SEQ ID NO: 268), and a CDRL3 sequence including QQYLYHPAT (SEQ ID NO: - 269). In particular embodiments, the PD-L1 binding domain includes a variable heavy chain including a CDRH1 sequence including SGFTFSDSWIH (SEQ ID NO: 270), a CDRH2 sequence including WISPYGGSTYYADSVKG (SEQ ID NO: 271), and a CDRH3 sequence including RHWPGGFDY (SEQ ID NO: 272).

In particular embodiments, the PD-L1 binding domain includes a variable light chain including a CDRL1 sequence including TGTSSDVGGYNYVS (SEQ ID NO: 273), a CDRL2 sequence including DVSNRPS (SEQ ID NO: 274), and a CDRL3 sequence including SSYTSSSTRV (SEQ ID NO: 275). In particular embodiments, the PD-L1 binding domain includes a variable heavy chain including a CDRH1 sequence including SGFTFSSYIMM (SEQ ID NO: 276), a CDRH2 sequence including SIYPSGGITFYADTVKG (SEQ ID NO: 277), and a CDRH3 sequence including IKLGTVTTVDY (SEQ ID NO: 259).

In particular embodiments, the PD-L1 binding domain of at least one BS-BDC in a group is a human or humanized binding domain (e.g., scFv) including a variable light chain including a CDRL1 sequence including RASQSVSSYL (SEQ ID NO: 82), a CDRL2 sequence including DASNRAT (SEQ ID NO: 83), and a CDRL3 sequence including QQRSNWPRT (SEQ ID NO: 84). In particular embodiments, the cancer antigen epitope binding domain of at least one BS-BDC in a group is a human or humanized binding domain (e.g., scFv) including a variable heavy chain including a CDRH1 sequence including DYGFS (SEQ ID NO: 85), a CDRH2 sequence including WITAYNGNTNYAQKLQG (SEQ ID NO: 86), and a CDRH3 sequence including DYFYGMDY (SEQ ID NO: 87). These reflect CDR sequences of the 3G10 antibody. Numerous additional sequences that bind PD-L1 are described in, for example, US 2016/0222117.

In particular embodiments, the cancer antigen epitope binding domains present within a BS-BDC group each target a different CD123 epitope. In particular embodiments, the cancer antigen epitope binding domain of at least one BS-BDC in a group is a human or humanized binding domain (e.g., scFv) including the CDRs of the anti-CD123 7G3 antibody. In particular embodiments, the cancer antigen epitope binding domain of at least one BS-BDC in a group is a human or humanized binding domain (e.g., scFv) including a variable light chain including a CDRL1 sequence including RASESVDNYGNTFMH (SEQ ID NO: 88), a CDRL2 sequence including RASNLES (SEQ ID NO: 89), and a CDRL3 sequence including QQSNEDPPT (SEQ ID NO: 90). In particular embodiments, the cancer antigen epitope binding domain of at least one BS-BDC in a group is a human or humanized binding domain (e.g., scFv) including a variable heavy chain including a CDRH1 sequence including NYGMN (SEQ ID NO: 91), a CDRH2 sequence including WINTYTGESTYSADFKG (SEQ ID NO: 92), and a CDRH3 sequence including SGGYDPMDY (SEQ ID NO: 93). These reflect CDR sequences of antibody 32716 described in US Patent Number 8,163,279.

In particular aspects of the disclosure, the cancer antigen epitope binding domain of at least one BS-BDC in a group is a human or humanized binding domain (e.g., scFv) including a variable light chain including a CDRL1 sequence including RSNKSLLHSNGNTYLY (SEQ ID NO: 94), a CDRL2 sequence including RMSNLAS (SEQ ID NO: 95), and a CDRL3 sequence including MQHLEYPYT (SEQ ID NO: 96). In particular embodiments, the cancer antigen epitope binding domain of at least one BS-BDC in a group is a human or humanized binding domain (e.g., scFv) including a variable heavy chain including a CDRH1 sequence including NYWMN (SEQ ID NO: 97), a CDRH2 sequence including RIDPSDSESHYNQKFKD (SEQ ID NO: 98), and a CDRH3 sequence including YDYDDTMDY (SEQ ID NO: 99). These reflect CDR sequences of antibody 32703 described in US Patent Number 8,163,279.

Immune Cell Activating Epitopes. Immune cells that can be targeted for localized activation by SMITEs of the current disclosure include, for example, T cells, natural killer (NK) cells, and macrophages.

T-cell activation can be mediated by two distinct signals: those that initiate antigen-dependent primary activation and provide a T-cell receptor like signal (primary cytoplasmic signaling sequences) and those that act in an antigen- independent manner to provide a secondary or co-stimulatory signal (secondary cytoplasmic signaling sequences). BS-BDC groups disclosed herein can target any combination of T cell activating epitopes that upon binding induce T-cell activation. Examples of such T cell activating epitopes are on T cell markers including CD2, CD3, CD7, CD27, CD28, CD30, CD40, CD83, 4-1BB (CD 137), OX40, lymphocyte function-associated antigen-1 (LFA-1), LIGHT, NKG2C, and B7-H3. T cell suppressive receptors that can be blocked include 4-1BB, PD-1, LAG3, TIM-3, BTLA, CTLA-4, and CD200.

CD3 is a primary signal transduction element of T cell receptors. CD3 is composed of a group of invariant proteins called gamma (γ), delta (Δ), epsilon (Σ), zeta (Z) and eta (H) chains. The γ, Δ, and Σ chains are structurally-related, each containing an Ig-like extracellular constant domain followed by a transmembrane region and a cytoplasmic domain of more than 40 amino acids. The Z and H chains have a distinctly different structure: both have a very short extracellular region of only 9 amino acids, a transmembrane region and a long cytoplasmic tail including 113 and 115 amino acids in the Z and H chains, respectively. The invariant protein chains in the CD3 complex associate to form noncovalent heterodimers of the Σ chain with a γ chain (Σγ) or with a Δ chain (ΣΔ) or of the Z and H chain (ZH), or a disulfide-linked homodimer of two Z chains (ZZ). 90% of the CD3 complex incorporate the ZZ homodimer.

The cytoplasmic regions of the CD3 chains include a motif designated the immunoreceptor tyrosine-based activation motif (ITAM). This motif is found in a number of other receptors including the Ig-α/Ig-β heterodimer of the B-cell receptor complex and Fc receptors for IgE and IgG. The ITAM sites associate with cytoplasmic tyrosine kinases and participate in signal transduction following TCR-mediated triggering. In CD3, the γ, Δ and Σ chains each contain a single copy of ITAM, whereas the Z and H chains harbor three ITAMs in their long cytoplasmic regions. Indeed, the Z and H chains have been ascribed a major role in T cell activation signal transduction pathways.

CD3 is expressed on all mature T cells. In particular embodiments, the CD3 binding domain (e.g., scFv) is derived from the OKT3 antibody (the same as the one utilized in blinatumomab). The OKT3 antibody is described in detail in U.S. Patent No. 5,929,212. It includes a variable light chain including a CDRL1 sequence including SASSSVSYMN (SEQ ID NO: 100), a CDRL2 sequence including RWIYDTSKLAS (SEQ ID NO: 101), and a CDRL3 sequence including QQWSSNPFT (SEQ ID NO: 102). In particular embodiments, the CD3 T cell activating epitope binding domain of at least one BS-BDC in a group is a human or humanized binding domain (e.g., scFv) including a variable heavy chain including a CDRH1 sequence including KASGYTFTRYTMH (SEQ ID NO: 103), a CDRH2 sequence including INPSRGYTNYNQKFKD (SEQ ID NO: 104), and a CDRH3 sequence including YYDDHYCLDY (SEQ ID NO: 105).

The following sequence is an scFv derived from OKT3 which retains the capacity to bind CD3: QVQLQQSGAELARPGASVKMSCKASGYTFTRYTM HWVKQRPGQGLEWIGYI N PSRGY TNYNQKFKDKATLTTDKSSSTAYMQLSSLTSEDSAVYYCARYYDDHYCLDYWGQGTTLTVSS SGGGGSGGGGSGGGGSQIVLTQSPAIMSASPGEKVTMTCSASSSVSYMNWYQQKSGTSPKR WIYDTSKLASGVPAHFRGSGSGTSYSLTISGMEAEDAATYYCQQWSSNPFTFGSGTKLEINR (SEQ ID NO: 106). It may also be used as a CD3 binding domain.

In particular embodiments, the CD3 T cell activating epitope binding domain of at least one BS-BDC in a group is a human or humanized binding domain (e.g., scFv) including a variable light chain including a CDRL1 sequence including QSLVHNNGNTY (SEQ ID NO: 107), a CDRL2 sequence including KVS, and a CDRL3 sequence including GQGTQYPFT (SEQ ID NO: 109). In particular embodiments, the CD3 T cell activating epitope binding domain of at least one BS-BDC in a group is a human or humanized binding domain (e.g., scFv) including a variable heavy chain including a CDRH1 sequence including GFTFTKAW (SEQ ID NO: 110), a CDRH2 sequence including IKDKSNSYAT (SEQ ID NO: 111), and a CDRH3 sequence including RGVYYALSPFDY (SEQ ID NO: 112). These reflect CDR sequences of the 20G6-F3 antibody.

In particular embodiments, the CD3 T cell activating epitope binding domain of at least one BS-BDC in a group is a human or humanized binding domain (e.g., scFv) including a variable light chain including a CDRL1 sequence including QSLVHDNGNTY (SEQ ID NO: 113), a CDRL2 sequence including KVS, and a CDRL3 sequence including GQGTQYPFT (SEQ ID NO: 115). In particular embodiments, the CD3 T cell activating epitope binding domain of at least one BS-BDC in a group is a human or humanized binding domain (e.g., scFv) including a variable heavy chain including a CDRH1 sequence including GFTFSNAW (SEQ ID NO: 116), a CDRH2 sequence including IKARSNNYAT (SEQ ID NO: 117), and a CDRH3 sequence including RGTYYASKPFDY (SEQ ID NO: 118). These reflect CDR sequences of the 4B4-D7antibody.

In particular embodiments, the CD3 T cell activating epitope binding domain of at least one BS-BDC in a group is a human or humanized binding domain (e.g., scFv) including a variable light chain including a CDRL1 sequence including QSLEHNNGNTY (SEQ ID NO: 119), a CDRL2 sequence including KVS, and a CDRL3 sequence including GQGTQYPFT (SEQ ID NO: 121). In particular embodiments, the CD3 T cell activating epitope binding domain of at least one BS-BDC in a group is a human or humanized binding domain (e.g., scFv) including a variable heavy chain including a CDRH1 sequence including GFTFSNAW (SEQ ID NO: 122), a CDRH2 sequence including IKDKSNNYAT (SEQ ID NO: 123), and a CDRH3 sequence including RYVHYGIGYAMDA (SEQ ID NO: 124). These reflect CDR sequences of the 4E7-C9 antibody.

In particular embodiments, the CD3 T cell activating epitope binding domain of at least one BS-BDC in a group is a human or humanized binding domain (e.g., scFv) including a variable light chain including a CDRL1 sequence including QSLVHTNGNTY (SEQ ID NO: 125), a CDRL2 sequence including KVS, and a CDRL3 sequence including GQGTHYPFT (SEQ ID NO: 127). In particular embodiments, the CD3 T cell activating epitope binding domain of at least one BS-BDC in a group is a human or humanized binding domain (e.g., scFv) including a variable heavy chain including a CDRH1 sequence including GFTFTNAW (SEQ ID NO: 128), a CDRH2 sequence including KDKSNNYAT (SEQ ID NO: 129), and a CDRH3 sequence including RYVHYRFAYALDA (SEQ ID NO: 130). These reflect CDR sequences of the 18F5-H10 antibody.

Additional examples of anti-CD3 antibodies, binding domains, and CDRs can be found in WO2016/116626. TR66 may also be used.

CD28 is a surface glycoprotein present on 80% of peripheral T cells in humans, and is present on both resting and activated T cells. CD28 binds to B7-1 (CD80) and B7-2 (CD86) and is the most potent of the known co-stimulatory molecules (June et al., Immunol. Today 15:321 (1994); Linsley et al., Ann. Rev. Immunol. 11:191 (1993)). In particular embodiments, the CD28 binding domain (e.g., scFv) is derived from CD80, CD86 or the 9D7 antibody. Additional antibodies that bind CD28 include 9.3, KOLT-2, 15E8, 248.23.2, and EX5.3D10. Further, 1YJD provides a crystal structure of human CD28 in complex with the Fab fragment of a mitogenic antibody (5.11A1). In particular embodiments, antibodies that do not compete with 9D7 are selected.

In particular embodiments at least one BS-BDC within a group binds an epitope of CD28. In particular embodiments, the CD28 binding domain includes the CDRs of the TGN1412 antibody. In particular embodiments, the CD28 binding domain including a variable light chain including a CDRL1 sequence including HASQNIYVWLN (SEQ ID NO: 131), a CDRL2 sequence including KASNLHT (SEQ ID NO: 132), and a CDRL3 sequence including QQGQTYPYT (SEQ ID NO: 133). In particular embodiments, the CD28 binding domain including a variable heavy chain including a CDRH1 sequence including SYYIH (SEQ ID NO: 134), a CDRH2 sequence including CIYPGNVNTNYNEKFKD (SEQ ID NO: 135), and a CDRH3 sequence including SHYGLDWNFDV (SEQ ID NO: 136).

In particular aspects of the disclosure at least one BS-BDC within a group binds an epitope of CD80/CD86. CD80 (also called B7-1, UniProt ID No. P33681, SEQ ID NO: 137) and CD86 (also called B7-2, UniProt ID No. P42081, SEQ ID NO: 138) both provide costimulatory signals for T-cell activation and survival. In particular embodiments a CD80/CD86 binding domain (e.g., scFv) is derived from one or more monoclonal antibodies described in U.S. Patent No. 7,531,175. In particular embodiments, the CD80/CD86 binding domain includes a variable light chain including a CDRL1 sequence including SVSSSISSSNLH (SEQ ID NO: 139), a CDRL2 sequence including GTSNLAS (SEQ ID NO: 140), and a CDRL3 sequence including QQWSSYPLT (SEQ ID NO: 141). In particular embodiments, the CD80/CD86 binding domain includes a variable heavy chain including a CDRH1 sequence including DYYMH (SEQ ID NO: 142), a CDRH2 sequence including WIDPENGNTLYDPKFQG (SEQ ID NO: 143), and a CDRH3 sequence including EGLFFAY (SEQ ID NO: 144).

Activated T-cells express 4-1BB (CD137). T-cells can further be classified into helper cells (CD4+ T-cells) and cytotoxic T-cells (CTLs, CD8+ T-cells), which include cytolytic T-cells. T helper cells assist other white blood cells in immunologic processes, including maturation of B cells into plasma cells and activation of cytotoxic T-cells and macrophages, among other functions. These cells are also known as CD4+ T-cells because they express the CD4 protein on their surface. Helper T-cells become activated when they are presented with peptide antigens by MHC class II molecules that are expressed on the surface of antigen presenting cells (APCs). Once activated, they divide rapidly and secrete small proteins called cytokines that regulate or assist in the active immune response.

Particular aspects of the disclosure can include activating CD4 T cells by binding CD3, TLR2 or CD28 and/or by blocking the suppression of CD4 T cells by binding 4-1BB, PD-1, LAG3, TIM-3, BTLA, CTLA-4, CD200, and/or VISTA.

TLR2 (UniProt ID No. 060603, SEQ ID NO: 145) is involved in the innate immune response to bacterial lipoproteins and other microbial cell wall components. In particular embodiments, the TLR2 binding domain is derived from an anti-TLR2 antibody. Commercially available anti-TLR2 antibodies include anti-hTLR2-lgA and mAb-hTLR2 (both available from Invivogen)

In particular aspects of the disclosure at least one BS-BDC within a group binds an epitope of co-stimulatory receptor 4-1BB. 4-1BB, also called CD137 or TNFSF9 (UniProt ID No. Q07011, SEQ ID NO: 146) is a T-cell co-stimulatory receptor. In particular embodiments a 4-1BB binding domain (e.g., scFv) is derived from a monoclonal antibody described in U.S. Patent Number 9,382,328B2. In particular embodiments, the 4-1BB binding domain includes a variable light chain including a CDRL1 sequence including RASQSVS (SEQ ID NO: 147), a CDRL2 sequence including ASNRAT (SEQ ID NO: 148), and a CDRL3 sequence including QRSNWPPALT (SEQ ID NO: 149). In particular embodiments, the 4-1BB binding domain includes a variable heavy chain including a CDRH1 sequence including YYWS (SEQ ID NO: - 150), a CDRH2 sequence including INH, and a CDRH3 sequence including YGPGNYDWYFDL (SEQ ID NO: 152).

In particular aspects of the disclosure, the 4-1BB binding domain includes a variable light chain including a CDRL1 sequence including SGDNIGDQYAH (SEQ ID NO: 261), a CDRL2 sequence including QDKNRPS (SEQ ID NO: 262), and a CDRL3 sequence including ATYTGFGSLAV (SEQ ID NO: 263). In particular aspects of the disclosure, the 4-1BB binding domain includes a variable heavy chain including a CDRH1 sequence including GYSFSTYWIS (SEQ ID NO: 264), a CDRH2 sequence including KIYPGDSYTNYSPS (SEQ ID NO: 265), and a CDRH3 sequence including GYGIFDY (SEQ ID NO: 266).

In particular aspects of the disclosure at least one BS-BDC within a group binds an epitope of programmed cell death protein 1 (PD-1). PD-1, also called CD279 (UniProt ID No. Q15116, SEQ ID NO: 153) is an inhibitory cell surface receptor involved in regulating the T-cell immune response. In particular embodiments a PD-1 binding domain (e.g., scFv) is derived from a monoclonal antibody described in U.S. Patent Publication 2011/0271358. In particular embodiments, the PD-1 binding domain includes a variable light chain including a CDRL1 sequence including RASQSVSTSGYSYMH (SEQ ID NO: 154), a CDRL2 sequence including FGSNLES (SEQ ID NO: 155), and a CDRL3 sequence including QHSWEIPYT (SEQ ID NO: - 156). In particular embodiments, the PD-1 binding domain includes a variable heavy chain including a CDRH1 sequence including SSWIH (SEQ ID NO: 157), a CDRH2 sequence including YIYPSTGFTEYNQKFKD (SEQ ID NO: 158), and a CDRH3 sequence including WRDSSGYHAMDY (SEQ ID NO: 159).

In particular embodiments, a PD-1 binding domain (e.g., scFv) is derived from a monoclonal antibody described in U.S. Patent Application 20090217401A1. In particular embodiments, the PD-1 binding domain includes a variable light chain including a CDRL1 sequence including RASQSVSSYLA (SEQ ID NO: 160), a CDRL2 sequence including DASNRAT (SEQ ID NO: 161), and a CDRL3 sequence including QQSSNWPRT (SEQ ID NO: - 162). In particular embodiments, the PD-1 binding domain includes a variable heavy chain including a CDRH1 sequence including NSGMH (SEQ ID NO: 163), a CDRH2 sequence including VLWYDGSKRYYADSVKG (SEQ ID NO: 164), and a CDRH3 sequence including NDDY (SEQ ID NO: 165).

In particular aspects of the disclosure at least one BS-BDC within a group binds an epitope of lymphocyte activation gene 3 protein (LAG3). LAG3, also called CD223 (UniProt ID No. P18627, SEQ ID NO: 166) binds to HLA classs-II antigens and is involved in activation of lymphocytes. In particular embodiments a LAG3 binding domain (e.g., scFv) is derived from a monoclonal antibody described in PCT Patent Publication WO/2014/008218. In particular embodiments, the LAG3 binding domain includes a variable light chain including a CDRL1 sequence including RASQSISSYLA (SEQ ID NO: 167), a CDRL2 sequence including of DASNRAT (SEQ ID NO: 168), and a CDRL3 sequence including QQRSNWPLT (SEQ ID NO: - 169). In particular embodiments, the LAG3 binding domain includes a variable heavy chain including a CDRH1 sequence including DYYWN (SEQ ID NO: 170), a CDRH2 sequence including EINHRGSTNSNPSLKS (SEQ ID NO: 171), and a CDRH3 sequence including GYSDYEYNWFDP (SEQ ID NO: 172).

In particular aspects of the disclosure at least one BS-BDC within a group binds an epitope of T-cell immunoglobulin mucin receptor 3 (TIM-3). TIM-3, also known as HAVcr-2 or TIMD-3 (UniProt ID No. Q9TDQ0; SEQ ID NO: 173) is a cell surface receptor that plays an inhibitory role in innate and adaptive immune responses. In particular embodiments a TIM-3 binding domain (e.g., scFv) is derived from a monoclonal antibody described in U.S. Patent Publication 2015/0218274. In particular embodiments, the TIM-3 binding domain includes a variable light chain including a a CDRL1 sequence including SESVEYYGTSL (SEQ ID NO: 174), a CDRL2 sequence including AAS, and a CDRL3 sequence including SRKDPS (SEQ ID NO: 176). In particular embodiments, the TIM-3 binding domain includes a variable heavy chain including a CDRH1 sequence including GYTFTSY (SEQ ID NO: 177), a CDRH2 sequence including YPGNGD (SEQ ID NO: 178), and a CDRH3 sequence including VGGAFPMDY (SEQ ID NO: 179).

In particular aspects of the disclosure at least one BS-BDC within a group binds an epitope of B- and T-lymphocyte attenuator (BTLA). BTLA, also known as CD272 (UniProt ID No. Q7Z6A9, SEQ ID NO: 180), is an inhibitory receptor that inhibits the immune response of lymphocytes. In particular embodiments a BTLA binding domain (e.g., scFv) is derived from one or more monoclonal antibodies described in U.S. Patent Publication 2012/0288500. In particular embodiments, the BTLA binding domain includes a variable light chain including a CDRL1 sequence including RASQSVSSSYLA (SEQ ID NO: 181), a CDRL2 sequence including GASSRAT (SEQ ID NO: 182), and a CDRL3 sequence including QQYGSSIT (SEQ ID NO: - 183). In particular embodiments, the BTLA binding domain includes a variable heavy chain including a CDRH1 sequence including TIGVGVN (SEQ ID NO: 184), a CDRH2 sequence including LlYWDDDKRYSPSLKR (SEQ ID NO: 185), and a CDRH3 sequence including SGITEVRGVIIHYYGMDV (SEQ ID NO: 186).

In particular embodiments, the BTLA binding domain includes a variable light chain including a CDRL1 sequence including RASQSVSSSYLA (SEQ ID NO: 187), a CDRL2 sequence including of GASSRAT (SEQ ID NO: 188), and a CDRL3 sequence including QQYGSSPPIT (SEQ ID NO: 189). In particular embodiments, the BTLA binding domain includes a variable heavy chain including a CDRH1 sequence including TSGMCVS (SEQ ID NO: 190), a CDRH2 sequence including LIDWDDVKYYSSSLKT (SEQ ID NO: 191), and a CDRH3 sequence including IRFTMFRGVYYYYYGLDV (SEQ ID NO: 192).

In particular aspects of the disclosure at least one BS-BDC within a group binds an epitope of cytotoxic T-lymphocyte protein 5 (CTLA-4). CTLA-4, also known as CD152 (UniProt ID No. P16410, SEQ ID NO: 193), is an inhibitory receptor that is a major negative regulator of the T-cell response. In particular embodiments a CTLA-4 binding domain (e.g., scFv) is derived from a monoclonal antibody described in U.S. Patent No. 6,984,720. In particular embodiments, the CTLA-4 binding domain includes the CDRs of the Hu26B antibody. In particular embodiments, the CTLA-4 binding domain includes a variable light chain including a CDRL1 sequence including RASQSVGSSYLA (SEQ ID NO: 194), a CDRL2 sequence including GAFSRAT (SEQ ID NO: 195), and a CDRL3 sequence including QQYGSSPWT (SEQ ID NO: - 196). In particular embodiments, the CTLA-4 binding domain includes a variable heavy chain including a CDRH1 sequence including SYTMH (SEQ ID NO: 197), a CDRH2 sequence including FISYDGNNKYYADSVKG (SEQ ID NO: 198), and a CDRH3 sequence including TGWLGPFDY (SEQ ID NO: 199).

In particular embodiments, the CTLA-4 binding domain includes a variable light chain including a CDRL1 sequence including RASQGISSWLA (SEQ ID NO: 200), a CDRL2 sequence including AASSLQS (SEQ ID NO: 201), and a CDRL3 sequence including QQYNSYPPT (SEQ ID NO: 202). In particular embodiments, the CTLA-4 binding domain includes a variable heavy chain including a CDRH1 sequence including SYGMH (SEQ ID NO: 203), a CDRH2 sequence including VIWYDGSNKYYADSVKG (SEQ ID NO: 204), and a CDRH3 sequence including APNYIGAFDV (SEQ ID NO: 205).

In particular embodiments, the CTLA-4 binding domain includes a variable light chain including a CDRL1 sequence including SATSSITYMS (SEQ ID NO: 206), a CDRL2 sequence including DTSNLAS (SEQ ID NO: 207), and a CDRL3 sequence including QQWSSYPLT (SEQ ID NO: 208). In particular embodiments, the CTLA-4 binding domain includes a variable heavy chain including a CDRH1 sequence including SYGVY (SEQ ID NO: 209), a CDRH2 sequence including VIWAGGTTNYNSALMS (SEQ ID NO: 210), and a CDRH3 sequence including GPPHAMMKRGYAMDY (SEQ ID NO: 211). These reflect CDRs sequences described in US Patent Application US20020039581A1.

In particular aspects of the disclosure at least one BS-BDC within a group binds an epitope of CD200. CD200 (also known as ox-2 membrane glycoprotein, UniProt ID No. P41217, SEQ ID NO: 212) is a protein that can deliver inhibitory signals to immune cells. In particular embodiments a CD200 binding domain (e.g., scFv) is derived from one or more monoclonal antibodies described in U.S. Patent Publication 2013/0189258. In particular embodiments, the CD200 binding domain includes a variable light chain including a CDRL1 sequence including RASESVDSYGNSFMH (SEQ ID NO: 213), a CDRL2 sequence including RASNLES (SEQ ID NO: 214), and a CDRL3 sequence including QQSNEDPRT (SEQ ID NO: 215). In particular embodiments, the CD200 binding domain includes a variable heavy chain including a CDRH1 sequence including GFTFSGFAMS (SEQ ID NO: 216), a CDRH2 sequence including SISSGGTTYYLDSVKG (SEQ ID NO: 217), and a CDRH3 sequence including GNYYSGTSYDY (SEQ ID NO: 218).

In particular aspects of the disclosure at least one BS-BDC within a group binds an epitope of V-type immunoglobulin domain-containing suppressor of T-cell activation precursor (VISTA; NP_071436.1; SEQ ID NO: 219). Binding domains for VISTA can be derived from antibodies available from, for example, R&D Systems, LifeSpan Biosciences, Invitrogen, BioLegend, BD Biosciences, and Abcam. In particular embodiments a VISTA binding domain (e.g., scFv) is derived from one or more monoclonal antibodies described in U.S. Patent Application 2017/0051061 or International Patent Publication WO2015097536A2. In particular embodiments, a VISTA binding domain (e.g., scFv) is derived from the antibody JNJ-61610588, which binds to and inhibits VISTA signaling. In particular embodiments, the VISTA binding domain includes a variable light chain including a CDRL1 sequence including GGTFSSY (SEQ ID NO: 220), a CDRL2 sequence including IIPIFGT (SEQ ID NO: 221), and a CDRL3 sequence including ARSSYGW (SEQ ID NO: 222). In particular embodiments, the VISTA binding domain includes a variable heavy chain including a CDRH1 sequence including QSIDTR (SEQ ID NO: - 223), a CDRH2 sequence including SAS, and a CDRH3 sequence including QQSAYNP (SEQ ID NO: 225).

Cytotoxic T-cells destroy tumor cells. These cells are also known as CD8+ T-cells because they express the CD8 glycoprotein at their surface. These cells recognize their targets by binding to antigen associated with MHC class I, which is present on the surface of nearly every cell of the body. Particular embodiments can include activating CD8 T cells by binding CD3, CD28, or 4-1BB and/or by blocking the suppression of CD8 T cells by binding PD-1, LAG3, TIM-3, or VISTA.

Particular embodiments disclosed herein including binding domains that bind epitopes on CD8. In particular embodiments, the CD8 binding domain (e.g., scFv) is derived from the OKT8 antibody. For example, in particular embodiments, the CD8 T cell activating epitope binding domain of at least one BS-BDC in a group is a human or humanized binding domain (e.g., scFv) including a variable light chain including a CDRL1 sequence including RTSRSISQYLA (SEQ ID NO: 226), a CDRL2 sequence including SGSTLQS (SEQ ID NO: 227), and a CDRL3 sequence including QQHNENPLT (SEQ ID NO: 228). In particular embodiments, the CD8 T cell activating epitope binding domain of at least one BS-BDC in a group is a human or humanized binding domain (e.g., scFv) including a variable heavy chain including a CDRH1 sequence including GFNIKD (SEQ ID NO: 229), a CDRH2 sequence including RIDPANDNT (SEQ ID NO: 230), and a CDRH3 sequence including GYGYYVFDH (SEQ ID NO: 231). These reflect CDR sequences of the OKT8 antibody.

In particular embodiments, a binding domain is a single chain T-cell receptor (scTCR) including V_{α/β} and C_{α/β} chains (*e.g.*, V_{α}-C_{α}, V_{β}-C_{β}, V_{α}-V_{β}) or including V_{α}-C_{α}, V_{β}-C_{β}, V_{α}-V_{β} pair specific for a target epitope of interest. In particular embodiments, T cell activating epitope binding domains can be derived from or based on a V_{α}, V_{β}, C_{α}, or C_{β} of a known TCR (*e.g*., a high-affinity TCR).

In particular embodiments, T cell activating epitope binding domains include one or more (*e.g.,* 2, 3, 4, 5, 6, 7, 8, 9, 10) insertions, one or more (*e.g.,* 2, 3, 4, 5, 6, 7, 8, 9, 10) deletions, one or more (*e.g.,* 2, 3, 4, 5, 6, 7, 8, 9, 10) amino acid substitutions (*e.g.,* conservative amino acid substitutions or non-conservative amino acid substitutions), or a combination of the above-noted changes, when compared with the V_{α}, V_{β}, C_{α}, or C_{β} of a known TCR. An insertion, deletion or substitution may be anywhere in a V_{α}, V_{β}, C_{α}, or C_{β} region, including at the amino- or carboxy-terminus or both ends of these regions, provided that each CDR includes zero changes or at most one, two, or three changes and provided a binding domain including a modified V_{α}, V_{β}, C_{α}, or C_{β} region can still specifically bind its target with an affinity similar to wild type.

In particular embodiments natural killer cells (also known as NK cells, K cells, and killer cells) are targeted for localized activation by SMITEs. NK cells can induce apoptosis or cell lysis by releasing granules that disrupt cellular membranes, and can secrete cytokines to recruit other immune cells.

Examples of activating proteins expressed on the surface of NK cells include NKG2D, CD8, CD16, KIR2DL4, KIR2DS1, KIR2DS2, KIR3DS1, NKG2C, NKG2E, NKG2D, and several members of the natural cytotoxicity receptor (NCR) family. Examples of NCRs that activate NK cells upon ligand binding include NKp30, NKp44, NKp46, NKp80, and DNAM-1.

Examples of commercially available antibodies that bind to an NK cell receptor and induce and/or enhance activation of NK cells include: 5C6 and 1D11, which bind and activate NKG2D (available from BioLegend^{®} San Diego, CA); mAb 33, which binds and activates KIR2DL4 (available from BioLegend^{®}); P44-8, which binds and activates NKp44 (available from BioLegend^{®}); SK1, which binds and activates CD8; and 3G8 which binds and activates CD16.

In particular embodiments, the BS-BDCs can bind to and block an NK cell inhibitory receptor to enhance NK cell activation. Examples of NK cell inhibitory receptors that can be bound and blocked include KIR2DL1, KIR2DL2/3, KIR3DL1, NKG2A, and KLRG1. In particular embodiments, a binding domain that binds and blocks the NK cell inhibitory receptors KIR2DL1 and KIR2DL2/3 includes a variable light chain region of the sequence EIVLTQSPVTLSLSPGERATLSCRASQSVSSYLAWYQQKPGQAPRLLIYDASNRATGIPARFSG SGSGTDFTLTISSLEPEDFAVYYCQQRSNWMYTFGQGTKLEIKRT (SEQ ID NO: 232) and a variable heavy chain region of the sequence QVQLVQSGAEVKKPGSSVKVSCKA SGGTFSFYAISWVRQAPGQGLEWMGGFIPIFGAANYAQKFQGRVTITADESTSTAYMELSSLR SDDTAVYYCARIPSGSYYYDYDMDVWGQGTTVTVSS (SEQ ID NO: 233).

Additional NK cell activating antibodies are described in WO/2005/0003172 and US Patent No. 9,415,104.

In particular embodiments macrophages are targeted for localized activation by SMITEs. Macrophages are a type of leukocyte (or white blood cell) that can engulf and digest cells, cellular debris, and/or foreign substances in a process known as phagocytosis.

The BS-BDC groups can be designed to bind to a protein expressed on the surface of macrophages. Examples of activating proteins expressed on the surface of macrophages (and their precursors, monocytes) include CD11b, CD11c, CD64, CD68, CD119, CD163, CD206, CD209, F4/80, IFGR2 Toll-like receptors (TLRs) 1-9, IL-4Rα, and MARCO. Commercially available antibodies that bind to proteins expressed on the surface of macrophages include M1/70, which binds and activates CD11b (available from BioLegend^{®}); KP1, which binds and activates CD68 (available from ABCAM^{®}, Cambridge, United Kingdom); and ab87099, which binds and activates CD163 (available from ABCAM^{®}).

In particular aspects of the disclosureat least one BS-BDC within a group binds an epitope of CD40. CD40 (or Tumor necrosis factor receptor superfamily member 5, UniProt ID No. P25942, SEQ ID NO: 234) is a receptor that can transduce activating signals in macrophages. In particular embodiments, the CD40 binding domain is derived from the CD40-activating antibody CP-870,893.

In particular embodiments, examples of inhibitory proteins expressed by macrophages (and their precursors, monocytes) include programmed cell death ligands 1 and 2 (PD-L1 and PD-L2) and galectin 9 (Gal-9).

In particular aspects of the disclosure at least one BS-BDC within a group binds to and inhibits PD-L1. PD-L1 (also known as CD274 or B7-H1, UniProt ID No. Q9NZQ7, SEQ ID NO: 235) can inhibit T-cell proliferation and cytokine production. In particular embodiments, the PD-L1 binding domain can be derived from an anti-PD-L1 antibody. An example of a commercially available antibody that blocks PD-L1 is Nivolumab. An example of a neutralizing antibody that binds to and neutralizes PD-L1 is the monoclonal antibody 71213 (available from BPS Bioscience).

In particular aspects of the disclosure at least one BS-BDC within a group binds an epitope of PD-L2. PD-L2 (also known as CD273, UniProt ID No. Q9WUL5, SEQ ID NO: 236) can interact with TIM-3 and induce proliferation of regulatory T-cells, and induce apoptosis of cytotoxic T-cells. In particular embodiments, the PD-L2 binding domain is derived from an anti-PD-L2 antibody. An example of a commercially available PD-L2 antibody includes TY25 (ab21107, available from Abcam).

In particular aspects of the disclosure at least one BS-BDC within a group binds an epitope of Gal-9 (UniProt ID No. 000182, SEQ ID NO: 237) In particular embodiments, the Gal-9 binding domain can be derived from an anti-Gal-9 antibody that blocks binding to TIM-3. An example of a commercially available anti-Gal-9 antibody that blocks TIM-3 binding is 9M1-3 (available from Biolegend).

In particular embodiments, SMITEs can target a pathogen recognition receptor (PRR). PRRs are proteins or protein complexes that recognize a danger signal and activate and/or enhance the innate immune response. Examples of PRRs include the TLR4/MD-2 complex, which recognizes gram negative bacteria; Dectin-1 and Dectin-2, which recognize mannose moieties on fungus and other pathogens; TLR2/TLR6 or TLR2/TLR1 heterodimers, which recognize gram positive bacteria; TLR5, which recognizes flagellin; and TLR9 (CD289), which recognizes CpG motifs in DNA. In particular embodiments, BS-BDCs can bind and activate TLR4/MD-2, Dectin-1, Dectin-2, TRL2/TLR6, TLR2/TLR1, TLR5, and/or TLR9.

In particular embodiments, SMITEs can target the complement system. The complement system refers to an immune pathway that is induced by antigen-bound antibodies and involves signaling of complement proteins, resulting in immune recognition and clearance of the antibody-coated antigens. In particular embodiments, the BS-BDCs can bind complement-activating antibodies.

As indicated, in particular embodiments, a binding domain V_{H} region of the present disclosure can be derived from or based on a V_{H} of a known monoclonal antibody and can include one or more (*e.g.,* 2, 3, 4, 5, 6, 7, 8, 9, 10) insertions, one or more (*e.g.,* 2, 3, 4, 5, 6, 7, 8, 9, 10) deletions, one or more (*e.g.,* 2, 3, 4, 5, 6, 7, 8, 9, 10) amino acid substitutions (*e.g.,* conservative amino acid substitutions or non-conservative amino acid substitutions), or a combination of the above-noted changes, when compared with the V_{H} of a known monoclonal antibody. An insertion, deletion or substitution may be anywhere in the V_{H} region, including at the amino- or carboxy-terminus or both ends of this region, provided that each CDR includes zero changes or at most one, two, or three changes and provided a binding domain including the modified V_{H} region can still specifically bind its target with an affinity similar to the wild type binding domain.

In particular embodiments, a V_{L} region in a binding domain of the present disclosure is derived from or based on a V_{L} of a known monoclonal antibody and includes one or more (e.g., 2, 3, 4, 5, 6, 7, 8, 9, 10) insertions, one or more (*e.g*., 2, 3, 4, 5, 6, 7, 8, 9, 10) deletions, one or more (*e.g.,* 2, 3, 4, 5, 6, 7, 8, 9, 10) amino acid substitutions (*e.g.,* conservative amino acid substitutions), or a combination of the above-noted changes, when compared with the V_{L} of the known monoclonal antibody. An insertion, deletion or substitution may be anywhere in the V_{L} region, including at the amino- or carboxy-terminus or both ends of this region, provided that each CDR includes zero changes or at most one, two, or three changes and provided a binding domain including the modified V_{L} region can still specifically bind its target with an affinity similar to the wild type binding domain.

In particular embodiments, a binding domain includes or is a sequence that is at least 90%, at least 91%, at least 92%, at least 93%, at least 94%, at least 95%, at least 96%, at least 97%, at least 98%, at least 99%, at least 99.5%, or 100% identical to a known amino acid sequence of a light chain variable region (V_{L}) or to a heavy chain variable region (V_{H}), or both, wherein each CDR includes zero changes or at most one, two, or three changes, from a monoclonal antibody or fragment or derivative thereof that specifically binds to target of interest.

Particular embodiments include BS-BDC groups that bind: two different cancer antigen epitopes of ROR1 and CD3 and CD28. Particular embodiments include BS-BDC groups that bind: different cancer antigen epitopes and CD3, CD28, and CD137 (4-1BB). Particular embodiments include BS-BDC groups that bind: different cancer antigen epitopes and (i) two different epitopes on CD3 and (ii) CD28. Particular embodiments include BS-BDC groups that bind: different cancer antigen epitopes of ROR1 and (i) two different epitopes on CD28 and (ii) CD3.

Particular embodiments include BS-BDC that bind: ROR1/CD3; ROR1/CD28; CD33/CD3; CD19/CD3; CD123/CD3; CD33/CTLA-4; CD33/CD28; CD123/CD28; and PD-L1/CD28. Particular embodiments may utilize cancer antigen epitopes in combination with T cell activating epitopes as shown in the following Table 1:

**Table 1. Exemplary Targeted Cancer Antigen Epitope/T Cell Activating Epitope Combinations**

| | CD3 | CD28 | CD8 |
|---|---|---|---|
| ROR1-A | ROR1-A/CD3 | ROR1-A/CD28 | ROR1-A/CD8 |
| ROR1-a | ROR1-a/CD3 | ROR1-a/CD28 | ROR1-a/CD8 |
| ROR1-B | ROR1-B/CD3 | ROR1-B/CD28 | ROR1-B/CD8 |

In this table and elsewhere herein, ROR1-A can be interpreted synonymously with R11; ROR1-B can be interpreted synonymously with 2A2; and ROR1-a can be can be interpreted synonymously with R12. The R12 antibody targets an epitope that is different from and non-overlapping with the epitopes bound by R11 and 2A2. R11 and 2A2 target epitopes that are different and non-competing, so these two can bind ROR-1 simultaneously.

ROR1 epitopes in the preceding table may be replaced with epitopes from other cancer antigens disclosed herein (e.g., CD19, CD33, PSMA, mesothelin, CD123, PD-L1). Particular embodiments include ROR1/CD3 and ROR1/CD28 BS-BDC within a BS-BDC group. Particular embodiments include ROR1/CD28 and CD33/CD3 BS-BDC within a BS-BDC group. Particular embodiments include CD33/CD3 and PD-L1/CD28 BS-BDC within a BS-BDC group. Particular embodiments include CD19/CD3 and PD-L1/CD28 BS-BDC within a BS-BDC group. Particular embodiments include CD123/CD28 and CD123/CD3 BS-BDC within a BS-BDC group. Particular embodiments include CD33/CD3 and CD123/CD28 BS-BDC within a BS-BDC group.

In particular embodiments, each group of BS-BDC will target at least two different epitopes on the same cancer antigen. If additional epitopes are targeted, the additional epitopes can be on the same cancer antigen or can be on a different cancer antigen.

Particular examples of bispecific T-cell engaging antibodies that can be used within BS-BDC groups described herein include MDT000098 (SEQ ID NO: 238; bAb_2A2-CD28-His); MDT000099 (SEQ ID NO: 239; bAb_2A2-CD8-His); MDT000100 (SEQ ID NO: 240; bAb_R11-CD3-Myc-His); MDT000327 (SEQ ID NO: 241; bAb_R11-CD3-His (Version 2 of MDT000100)); MDT000346 (SEQ ID NO: 242; bAb_R11-CD28-His); MDT000320 (SEQ ID NO: 243; bAb_R12-CD3-His); MDT000347 (SEQ ID NO: 244; _bAb_R12-CD28-His); MDT000319 (SEQ ID NO: 245; _bAb_2A2-CD3-His); MDT000359 (SEQ ID NO: 246; _bAb_PDL1-CD28-His); MDT000479 (SEQ ID NO: 247; _scFv_CD28_TGN1412-His); MDT000480 (SEQ ID NO: 248; _scFv_PDL1_Tecentriq-His); MDT000244 (SEQ ID NO: 249; _bAb_Blincyto-His); MDT000245 (SEQ ID NO: 250; bAb_AMG330-His); MDT000470 (SEQ ID NO: 251; _bAb_Blincyto-CD28-His); a BS-BDC targeting ROR1 and 4-1BB (SEQ ID NO: 252; bAb_R12-CD137-His); ROR1/CD3 bispecific antibodies described in WO2014/167022; the CD19/CD3 antibody (Blinatumomab); the CD19/CD3 antibodies described in US 2016/0208001; and/or the Her2/CD3 antibodies described in US 2014/0302037 and US 2014/0308285, among others.

As indicated, binding domains of a BS-BDC may be joined through a linker. A linker is an amino acid sequence which can provide flexibility and room for conformational movement between the binding domains of a BS-BDC. Any appropriate linker may be used. Examples of linkers can be found in Chen et al., Adv Drug Deliv Rev. 2013 Oct 15; 65(10): 1357-1369. Linkers can be flexible, rigid, or semi-rigid, depending on the desired functional domain presentation to a target. Commonly used flexible linkers include Gly-Ser linkers such as GGSGGGSGGSG (SEQ ID NO: 120), GGSGGGSGSG (SEQ ID NO: 151) and GGSGGGSG (SEQ ID NO: 175). Additional examples include: GGGGSGGGGS (SEQ ID NO: 224); GGGSGGGS (SEQ ID NO: 108); and GGSGGS (SEQ ID NO: 114). Linkers that include one or more antibody hinge regions and/or immunoglobulin heavy chain constant regions, such as CH3 alone or a CH2CH3 sequence can also be used.

In some situations, flexible linkers may be incapable of maintaining a distance or positioning of binding domains needed for a particular use. In these instances, rigid or semi-rigid linkers may be useful. Examples of rigid or semi-rigid linkers include proline-rich linkers. In particular embodiments, a proline-rich linker is a peptide sequence having more proline residues than would be expected based on chance alone. In particular embodiments, a proline-rich linker is one having at least 30%, at least 35%, at least 36%, at least 39%, at least 40%, at least 48%, at least 50%, or at least 51% proline residues. Particular examples of proline-rich linkers include fragments of proline-rich salivary proteins (PRPs).

In particular embodiments, BS-BDC disclosed herein are formed using the Daedalus expression system as described in Pechman et al., Am J Physiol 294: R1234-R1239, 2008. The Daedalus system utilizes inclusion of minimized ubiquitous chromatin opening elements in transduction vectors to reduce or prevent genomic silencing and to help maintain the stability of decigram levels of expression. This system can bypass tedious and time-consuming steps of other protein production methods by employing the secretion pathway of serum-free adapted human suspension cell lines, such as 293 Freestyle. Using optimized lentiviral vectors, yields of 20-100 mg/l of correctly folded and post-translationally modified, endotoxin-free protein of up to 70 kDa in size, can be achieved in conventional, small-scale (100 ml) culture. At these yields, most proteins can be purified using a single size-exclusion chromatography step, immediately appropriate for use in structural, biophysical or therapeutic applications. Bandaranayake et al., Nucleic Acids Res., 2011 (Nov); 39(21). In some instances, purification by chromatography may not be needed due to the purity of manufacture according the methods described herein.

Particular embodiments utilize DNA constructs (e.g., chimeric genes, expression cassettes, expression vectors, recombination vectors, etc.) including a nucleic acid sequence encoding the protein or proteins of interest operatively linked to appropriate regulatory sequences. Such DNA constructs are not naturally-occurring DNA molecules and are useful for introducing DNA into host-cells to express selected proteins of interest.

Operatively linked refers to the linking of DNA sequences (including the order of the sequences, the orientation of the sequences, and the relative spacing of the various sequences) in such a manner that the encoded protein is expressed. Methods of operatively linking expression control sequences to coding sequences are well known in the art. See, e.g., Maniatis et al., Molecular Cloning: A Laboratory Manual, Cold Spring Harbor, N. Y., 1982; and Sambrook et al., Molecular Cloning: A Laboratory Manual, Cold Spring Harbor, N. Y., 1989.

Expression control sequences are DNA sequences involved in any way in the control of transcription or translation. Suitable expression control sequences and methods of making and using them are well known in the art. Expression control sequences generally include a promoter. The promoter may be inducible or constitutive. It may be naturally-occurring, may be composed of portions of various naturally-occurring promoters, or may be partially or totally synthetic. Guidance for the design of promoters is provided by studies of promoter structure, such as that of Harley and Reynolds, Nucleic Acids Res., 15, 2343-2361, 1987. Also, the location of the promoter relative to the transcription start may be optimized. See, e.g., Roberts et al., Proc. Natl. Acad. Sci. USA, 76:760-764, 1979.

The promoter may include, or be modified to include, one or more enhancer elements. In particular embodiments, the promoter will include a plurality of enhancer elements. Promoters including enhancer elements can provide for higher levels of transcription as compared to promoters that do not include them.

For efficient expression, the coding sequences can be operatively linked to a 3' untranslated sequence. In particular embodiments, the 3' untranslated sequence can include a transcription termination sequence and a polyadenylation sequence. The 3' untranslated region can be obtained, for example, from the flanking regions of genes.

In particular embodiments, a 5' untranslated leader sequence can also be employed. The 5' untranslated leader sequence is the portion of an mRNA that extends from the 5' CAP site to the translation initiation codon.

In particular embodiments, a "hisavi" tag can be added to the N-terminus or C-terminus of a gene by the addition of nucleotides coding for the Avitag amino acid sequence, "GLNDIFEAQKIEWHE" (SEQ ID NO: 126), as well as the 6xhistidine tag coding sequence "HHHHHH (SEQ ID NO: 260)". The Avitag avidity tag can be biotinylated by a biotin ligase to allow for biotin-avidin or biotin-streptavidin based interactions for protein purification, as well as for immunobiology (such as immunoblotting or immunofluorescence) using anti-biotin antibodies. The 6xhistidine tag allows for protein purification using Ni-2+ affinity chromatography.

Nucleic acid sequences encoding proteins disclosed herein can be derived by those of ordinary skill in the art. Nucleic acid sequences can also include one or more of various sequence polymorphisms, mutations, and/or sequence variants. In particular embodiments, the sequence polymorphisms, mutations, and/or sequence variants do not affect the function of the encoded protein. The sequences can also include degenerate codons of a native sequence or sequences that may be introduced to provide codon preference.

In some aspects, the DNA constructs can be introduced by transfection, a technique that involves introduction of foreign DNA into the nucleus of eukaryotic cells. In some aspects, the proteins can be synthesized by transient transfection (DNA does not integrate with the genome of the eukaryotic cells, but the genes are expressed for 24-96 hours). Various methods can be used to introduce the foreign DNA into the host-cells, and transfection can be achieved by chemical-based means including by the calcium phosphate, by dendrimers, by liposomes, and by the use of cationic polymers. Non-chemical methods of transfection include electroporation, sono-poration, optical transfection, protoplast fusion, impalefection, and hydrodynamic delivery. In some embodiments, transfection can be achieved by particle-based methods including gene gun where the DNA construct is coupled to a nanoparticle of an inert solid which is then "shot" directly into the target-cell's nucleus. Other particle-based transfection methods include magnet assisted transfection and impalefection.

In particular embodiments, the BS-BDC can be modified to produce an administration benefit. In particular embodiments, modified BS-BDC include those wherein one or more amino acids have been replaced with a non-amino acid component, or where the amino acid has been conjugated to a functional group or a functional group has been otherwise associated with an amino acid. The modified amino acid may be, e.g., a glycosylated amino acid, a PEGylated amino acid, a farnesylated amino acid, an acetylated amino acid, a biotinylated amino acid, an amino acid conjugated to a lipid moiety, or an amino acid conjugated to an organic derivatizing agent. Amino acid(s) can be modified, for example, co-translationally or post-translationally during recombinant production (e.g., N-linked glycosylation at N-X-S/T motifs during expression in mammalian cells) or modified by synthetic means. The modified amino acid can be within the sequence or at the terminal end of a sequence. Modifications also include nitrited constructs.

PEGylation particularly is a process by which polyethylene glycol (PEG) polymer chains are covalently conjugated to other molecules such as proteins. Several methods of PEGylating proteins have been reported in the literature. For example, N-hydroxy succinimide (NHS)-PEG was used to PEGylate the free amine groups of lysine residues and N-terminus of proteins; PEGs bearing aldehyde groups have been used to PEGylate the amino-termini of proteins in the presence of a reducing reagent; PEGs with maleimide functional groups have been used for selectively PEGylating the free thiol groups of cysteine residues in proteins; and site-specific PEGylation of acetyl-phenylalanine residues can be performed.

Covalent attachment of proteins to PEG has proven to be a useful method to increase the half-lives of proteins in the body (Abuchowski, A. et al., Cancer Biochem. Biophys.,1984, 7:175-186; Hershfield, M. S. et al., N. Engl. J. Medicine, 1987, 316:589-596; and Meyers, F. J. et al., Clin. Pharmacol. Ther., 1991, 49:307-313). The attachment of PEG to proteins not only protects the molecules against enzymatic degradation, but also reduces their clearance rate from the body. The size of PEG attached to a protein has significant impact on the half-life of the protein. The ability of PEGylation to decrease clearance is generally not a function of how many PEG groups are attached to the protein, but the overall molecular weight of the altered protein. Usually the larger the PEG is, the longer the *in vivo* half-life of the attached protein. In addition, PEGylation can also decrease protein aggregation (Suzuki et al., Biochem. Bioph. Acta vol. 788, pg. 248 (1984)), alter protein immunogenicity (Abuchowski et al.; J. Biol. Chem. vol. 252 pg. 3582 (1977)), and increase protein solubility as described, for example, in PCT Publication No. WO 92/16221).

Several sizes of PEGs are commercially available (Nektar Advanced PEGylation Catalog 2005-2006; and NOF DDS Catalogue Ver 7.1), which are suitable for producing proteins with targeted circulating half-lives. A variety of active PEGs have been used including mPEG succinimidyl succinate, mPEG succinimidyl carbonate, and PEG aldehydes, such as mPEG-propionaldehyde.

Sequence information provided by public databases can be used to identify additional gene and protein sequences that can be used with the systems and methods disclosed herein.

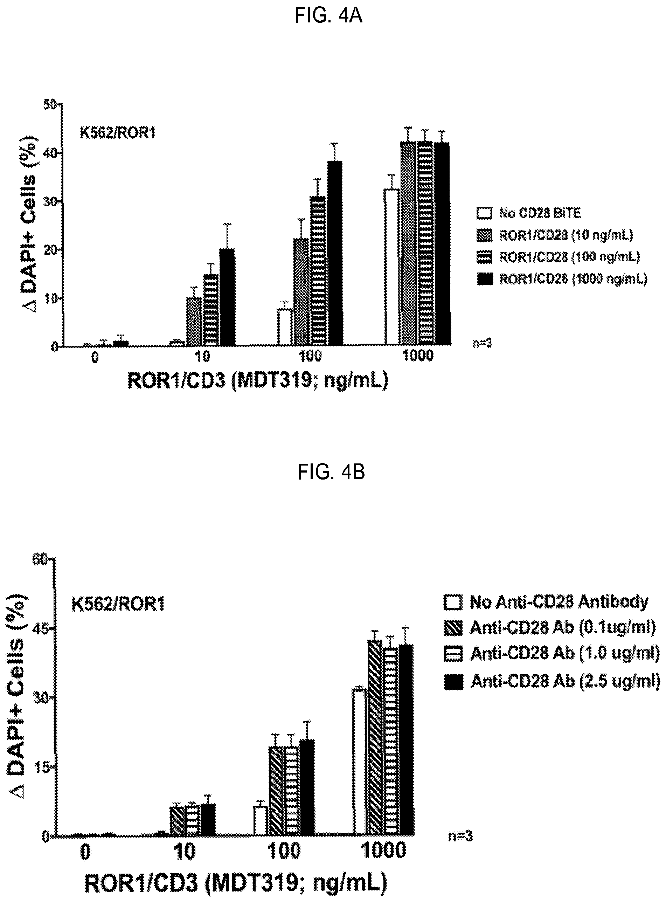

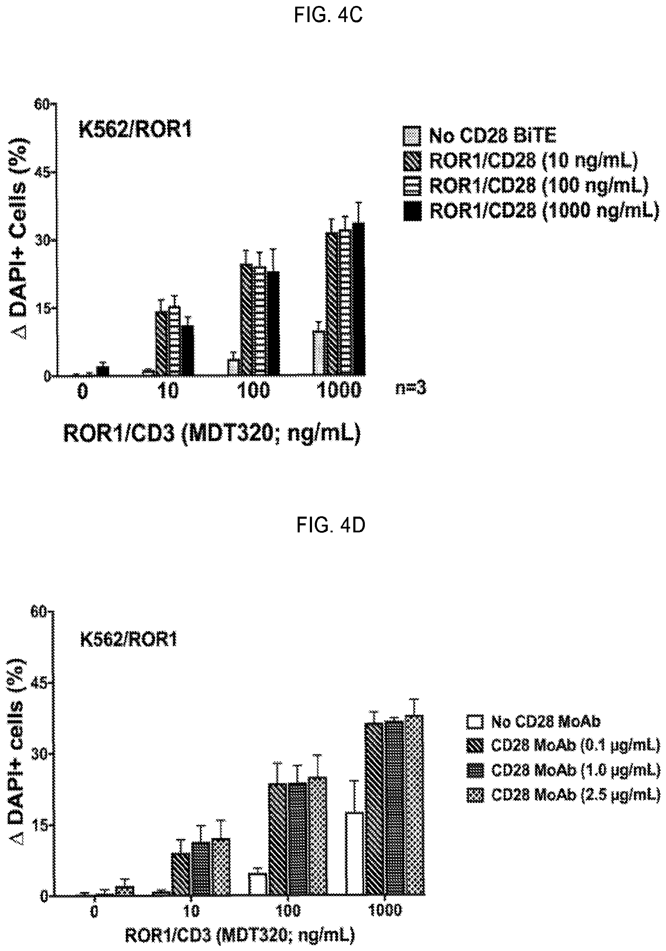

As indicated previously in relation to the discussion of binding domain sequences and encoding gene sequences, variants of the sequences disclosed and referenced herein are also included. Variants of proteins can include those having one or more conservative amino acid substitutions or one or more non-conservative substitutions that do not adversely affect the function of the protein in a measure described in for example, FIGs. 4-6. A "conservative substitution" involves a substitution found in one of the following conservative substitutions groups: Group 1: Alanine (Ala), Glycine (Gly), Serine (Ser), Threonine (Thr); Group 2: Aspartic acid (Asp), Glutamic acid (Glu); Group 3: Asparagine (Asn), Glutamine (Gln); Group 4: Arginine (Arg), Lysine (Lys), Histidine (His); Group 5: Isoleucine (Ile), Leucine (Leu), Methionine (Met), Valine (Val); and Group 6: Phenylalanine (Phe), Tyrosine (Tyr), Tryptophan (Trp).

Additionally, amino acids can be grouped into conservative substitution groups by similar function or chemical structure or composition (e.g., acidic, basic, aliphatic, aromatic, sulfur-containing). For example, an aliphatic grouping may include, for purposes of substitution, Gly, Ala, Val, Leu, and Ile. Other groups containing amino acids that are considered conservative substitutions for one another include: sulfur-containing: Met and Cysteine (Cys); acidic: Asp, Glu, Asn, and Gln; small aliphatic, nonpolar or slightly polar residues: Ala, Ser, Thr, Pro, and Gly; polar, negatively charged residues and their amides: Asp, Asn, Glu, and Gln; polar, positively charged residues: His, Arg, and Lys; large aliphatic, nonpolar residues: Met, Leu, Ile, Val, and Cys; and large aromatic residues: Phe, Tyr, and Trp. Additional information is found in Creighton (1984) Proteins, W.H. Freeman and Company.

As indicated elsewhere, variants of gene sequences can include codon optimized variants, sequence polymorphisms, splice variants, and/or mutations that do not affect the function of an encoded product to a statistically-significant degree.

Variants of the protein and nucleic acid sequences disclosed herein also include sequences with at least 70% sequence identity, 80% sequence identity, 85% sequence, 90% sequence identity, 95% sequence identity, 96% sequence identity, 97% sequence identity, 98% sequence identity, or 99% sequence identity to the protein and nucleic acid sequences described or disclosed herein.

"% sequence identity" refers to a relationship between two or more sequences, as determined by comparing the sequences. In the art, "identity" also means the degree of sequence relatedness between protein and nucleic acid sequences as determined by the match between strings of such sequences. "Identity" (often referred to as "similarity") can be readily calculated by known methods, including (but not limited to) those described in: Computational Molecular Biology (Lesk, A. M., ed.) Oxford University Press, NY (1988); Biocomputing: Informatics and Genome Projects (Smith, D. W., ed.) Academic Press, NY (1994); Computer Analysis of Sequence Data, Part I (Griffin, A. M., and Griffin, H. G., eds.) Humana Press, NJ (1994); Sequence Analysis in Molecular Biology (Von Heijne, G., ed.) Academic Press (1987); and Sequence Analysis Primer (Gribskov, M. and Devereux, J., eds.) Oxford University Press, NY (1992). Preferred methods to determine identity are designed to give the best match between the sequences tested. Methods to determine identity and similarity are codified in publicly available computer programs. Sequence alignments and percent identity calculations may be performed using the Megalign program of the LASERGENE bioinformatics computing suite (DNASTAR, Inc., Madison, Wisconsin). Multiple alignment of the sequences can also be performed using the Clustal method of alignment (Higgins and Sharp CABIOS, 5, 151-153 (1989) with default parameters (GAP PENALTY=10, GAP LENGTH PENALTY=10). Relevant programs also include the GCG suite of programs (Wisconsin Package Version 9.0, Genetics Computer Group (GCG), Madison, Wisconsin); BLASTP, BLASTN, BLASTX (Altschul, et al., J. Mol. Biol. 215:403-410 (1990); DNASTAR (DNASTAR, Inc., Madison, Wisconsin); and the FASTA program incorporating the Smith-Waterman algorithm (Pearson, Comput. Methods Genome Res., [Proc. Int. Symp.] (1994), Meeting Date 1992, 111-20. Editor(s): Suhai, Sandor. Publisher: Plenum, New York, N.Y.. Within the context of this disclosure it will be understood that where sequence analysis software is used for analysis, the results of the analysis are based on the "default values" of the program referenced. "Default values" will mean any set of values or parameters, which originally load with the software when first initialized.

BS-BDC can be formulated alone or in combination into compositions for administration to subjects. In particular embodiments, compositions include at least two BS-BDC disclosed herein formulated with a pharmaceutically acceptable carrier.

Salts and/or pro-drugs of BS-BDC can also be used.

A pharmaceutically acceptable salt includes any salt that retains the activity of the BS-BDC and is acceptable for pharmaceutical use. A pharmaceutically acceptable salt also refers to any salt which may form in vivo as a result of administration of an acid, another salt, or a prodrug which is converted into an acid or salt.

Suitable pharmaceutically acceptable acid addition salts can be prepared from an inorganic acid or an organic acid. Examples of such inorganic acids are hydrochloric, hydrobromic, hydroiodic, nitric, carbonic, sulfuric and phosphoric acid. Appropriate organic acids can be selected from aliphatic, cycloaliphatic, aromatic, arylaliphatic, heterocyclic, carboxylic and sulfonic classes of organic acids.

Suitable pharmaceutically acceptable base addition salts include metallic salts made from aluminum, calcium, lithium, magnesium, potassium, sodium and zinc or organic salts made from N,N'-dibenzylethylene-diamine, chloroprocaine, choline, diethanolamine, ethylenediamine, N-methylglucamine, lysine, arginine and procaine.

A prodrug includes an active ingredient which is converted to a therapeutically active compound after administration, such as by cleavage of a BS-BDC or by hydrolysis of a biologically labile group.

In particular embodiments, the compositions include BS-BDC of at least 0.1% w/v or w/w of the composition; at least 1% w/v or w/w of composition; at least 10% w/v or w/w of composition; at least 20% w/v or w/w of composition; at least 30% w/v or w/w of composition; at least 40% w/v or w/w of composition; at least 50% w/v or w/w of composition; at least 60% w/v or w/w of composition; at least 70% w/v or w/w of composition; at least 80% w/v or w/w of composition; at least 90% w/v or w/w of composition; at least 95% w/v or w/w of composition; or at least 99% w/v or w/w of composition.

Exemplary generally used pharmaceutically acceptable carriers include any and all absorption delaying agents, antioxidants, binders, buffering agents, bulking agents or fillers, chelating agents, coatings, disintegration agents, dispersion media, gels, isotonic agents, lubricants, preservatives, salts, solvents or co-solvents, stabilizers, surfactants, and/or delivery vehicles.

Exemplary antioxidants include ascorbic acid, methionine, and vitamin E.

Exemplary buffering agents include citrate buffers, succinate buffers, tartrate buffers, fumarate buffers, gluconate buffers, oxalate buffers, lactate buffers, acetate buffers, phosphate buffers, histidine buffers, and/or trimethylamine salts.

An exemplary chelating agent is EDTA.

Exemplary isotonic agents include polyhydric sugar alcohols including trihydric or higher sugar alcohols, such as glycerin, erythritol, arabitol, xylitol, sorbitol, or mannitol.

Exemplary preservatives include phenol, benzyl alcohol, meta-cresol, methyl paraben, propyl paraben, octadecyldimethylbenzyl ammonium chloride, benzalkonium halides, hexamethonium chloride, alkyl parabens such as methyl or propyl paraben, catechol, resorcinol, cyclohexanol, and 3-pentanol.

Stabilizers refer to a broad category of excipients which can range in function from a bulking agent to an additive which solubilizes the BS-BDC or helps to prevent denaturation or adherence to the container wall. Typical stabilizers can include polyhydric sugar alcohols; amino acids, such as arginine, lysine, glycine, glutamine, asparagine, histidine, alanine, ornithine, L-leucine, 2-phenylalanine, glutamic acid, and threonine; organic sugars or sugar alcohols, such as lactose, trehalose, stachyose, mannitol, sorbitol, xylitol, ribitol, myoinisitol, galactitol, glycerol, and cyclitols, such as inositol; PEG; amino acid polymers; sulfur-containing reducing agents, such as urea, glutathione, thioctic acid, sodium thioglycolate, thioglycerol, alpha-monothioglycerol, and sodium thiosulfate; low molecular weight polypeptides (i.e., <10 residues); proteins such as human serum albumin, bovine serum albumin, gelatin or immunoglobulins; hydrophilic polymers such as polyvinylpyrrolidone; monosaccharides such as xylose, mannose, fructose and glucose; disaccharides such as lactose, maltose and sucrose; trisaccharides such as raffinose, and polysaccharides such as dextran. Stabilizers are typically present in the range of from 0.1 to 10,000 parts by weight based on therapeutic weight.

The compositions disclosed herein can be formulated for administration by, for example, injection, inhalation, infusion, perfusion, lavage, or ingestion. The compositions disclosed herein can further be formulated for intravenous, intradermal, intraarterial, intranodal, intralymphatic, intraperitoneal, intralesional, intraprostatic, intravaginal, intrarectal, topical, intrathecal, intratumoral, intramuscular, intravesicular, oral and/or subcutaneous administration and more particularly by intravenous, intradermal, intraarterial, intranodal, intralymphatic, intraperitoneal, intralesional, intraprostatic, intravaginal, intrarectal, intrathecal, intratumoral, intramuscular, intravesicular, and/or subcutaneous injection.

For injection, compositions can be formulated as aqueous solutions, such as in buffers including Hanks' solution, Ringer's solution, or physiological saline. The aqueous solutions can include formulatory agents such as suspending, stabilizing, and/or dispersing agents. Alternatively, the formulation can be in lyophilized and/or powder form for constitution with a suitable vehicle, e.g., sterile pyrogen-free water, before use.

For oral administration, the compositions can be formulated as tablets, pills, dragees, capsules, liquids, gels, syrups, slurries, suspensions and the like. For oral solid formulations such as powders, capsules and tablets, suitable excipients include binders (gum tragacanth, acacia, cornstarch, gelatin), fillers such as sugars, e.g. lactose, sucrose, mannitol and sorbitol; dicalcium phosphate, starch, magnesium stearate, sodium saccharine, cellulose, magnesium carbonate; cellulose preparations such as maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP); granulating agents; and binding agents. If desired, disintegrating agents can be added, such as corn starch, potato starch, alginic acid, cross-linked polyvinylpyrrolidone, agar, or alginic acid or a salt thereof such as sodium alginate. If desired, solid dosage forms can be sugar-coated or enteric-coated using standard techniques. Flavoring agents, such as peppermint, oil of wintergreen, cherry flavoring, orange flavoring, etc. can also be used.

Compositions can be formulated as an aerosol. In particular embodiments, the aerosol is provided as part of an anhydrous, liquid or dry powder inhaler. Aerosol sprays from pressurized packs or nebulizers can also be used with a suitable propellant, e.g., dichlorodifluoromethane, trichlorofluoromethane, dichlorotetrafluoroethane, carbon dioxide or other suitable gas. In the case of a pressurized aerosol, a dosage unit may be determined by providing a valve to deliver a metered amount. Capsules and cartridges of gelatin for use in an inhaler or insufflator may also be formulated including a powder mix of BS-BDC and a suitable powder base such as lactose or starch.

Compositions can also be formulated as depot preparations. Depot preparations can be formulated with suitable polymeric or hydrophobic materials (for example as an emulsion in an acceptable oil) or ion exchange resins, or as sparingly soluble derivatives, for example, as a sparingly soluble salts.

Additionally, compositions can be formulated as sustained-release systems utilizing semipermeable matrices of solid polymers including at least one BS-BDC group. Various sustained-release materials have been established and are well known by those of ordinary skill in the art. Sustained-release systems may, depending on their chemical nature, release BS-BDC following administration for a few weeks up to over 100 days. Depot preparations can be administered by injection; parenteral injection; instillation; or implantation into soft tissues, a body cavity, or occasionally into a blood vessel with injection through fine needles.

Depot formulations can include a variety of bioerodible polymers including poly(lactide), poly(glycolide), poly(caprolactone) and poly(lactide)-co(glycolide) (PLG) of desirable lactide:glycolide ratios, average molecular weights, polydispersities, and terminal group chemistries. Blending different polymer types in different ratios using various grades can result in characteristics that borrow from each of the contributing polymers.

The use of different solvents (for example, dichloromethane, chloroform, ethyl acetate, triacetin, N-methyl pyrrolidone, tetrahydrofuran, phenol, or combinations thereof) can alter microparticle size and structure in order to modulate release characteristics. Other useful solvents include water, ethanol, dimethyl sulfoxide (DMSO), N-methyl-2-pyrrolidone (NMP), acetone, methanol, isopropyl alcohol (IPA), ethyl benzoate, and benzyl benzoate.

Exemplary release modifiers can include surfactants, detergents, internal phase viscosity enhancers, complexing agents, surface active molecules, co-solvents, chelators, stabilizers, derivatives of cellulose, (hydroxypropyl)methyl cellulose (HPMC), HPMC acetate, cellulose acetate, pluronics (e.g., F68/F127), polysorbates, Span^{®} (Croda Americas, Wilmington, Delaware), poly(vinyl alcohol) (PVA), Brij^{®} (Croda Americas, Wilmington, Delaware), sucrose acetate isobutyrate (SAIB), salts, and buffers.

Excipients that partition into the external phase boundary of microparticles such as surfactants including polysorbates, dioctylsulfosuccinates, poloxamers, PVA, can also alter properties including particle stability and erosion rates, hydration and channel structure, interfacial transport, and kinetics in a favorable manner.

Additional processing of the disclosed sustained release depot formulations can utilize stabilizing excipients including mannitol, sucrose, trehalose, and glycine with other components such as polysorbates, PVAs, and dioctylsulfosuccinates in buffers such as Tris, citrate, or histidine. A freeze-dry cycle can also be used to produce very low moisture powders that reconstitute to similar size and performance characteristics of the original suspension.

Any composition disclosed herein can advantageously include any other pharmaceutically acceptable carriers which include those that do not produce significantly adverse, allergic, or other untoward reactions that outweigh the benefit of administration. Exemplary pharmaceutically acceptable carriers and formulations are disclosed in Remington's Pharmaceutical Sciences, 18th Ed. Mack Printing Company, 1990. Moreover, formulations can be prepared to meet sterility, pyrogenicity, general safety, and purity standards as required by U.S. FDA Office of Biological Standards and/or other relevant foreign regulatory agencies.

In particular embodiments, BS-BDC compositions include immunogenic compositions. An immunogenic composition refers to a composition that stimulates an immune response in a subject. The immune response can be, for example, a T-cell response. A T-cell response can be detected, for example, by measuring production of cytokines, such as IL-2.

In particular embodiments, BS-BDC compositions include therapeutic compositions. A therapeutic composition refers to a composition that treats a subject. A treatment can be detected by a reduction in a subject's disease or symptoms as described elsewhere herein.

Kits. Also disclosed herein are kits including one or more containers including one or more of the BS-BDC and/or compositions described herein. Associated with such container(s) can be a notice in the form prescribed by a governmental agency regulating the manufacture, use, or sale of pharmaceuticals or biological products, which notice reflects approval by the agency of manufacture, use, or sale for human administration. In particular embodiments, BS-BDC groups within kits are chosen based on assessment of a particular subject's anticipated disease course. In particular embodiments, BS-BDC within kits are updated for a particular subject based on on-going assessments of the subject's current disease status.

Methods disclosed herein include treating subjects (humans, veterinary animals (dogs, cats, reptiles, birds, etc.) livestock (horses, cattle, goats, pigs, chickens, etc.) and research animals (monkeys, rats, mice, fish, etc.) with compositions disclosed herein. Treating subjects includes delivering therapeutically effective amounts. Therapeutically effective amounts include those that provide effective amounts, prophylactic treatments and/or therapeutic treatments.

An "effective amount" is the amount of a composition necessary to result in a desired physiological change in the subject. For example, an effective amount can provide an immunogenic effect. Effective amounts are often administered for research purposes. Effective amounts disclosed herein can cause a statistically-significant effect in an animal model or in vitro assay relevant to the assessment of a cancer's development or progression. An immunogenic composition can be provided in an effective amount, wherein the effective amount stimulates an immune response.

A "prophylactic treatment" includes a treatment administered to a subject who does not display signs or symptoms of a cancer or displays only early signs or symptoms of a cancer such that treatment is administered for the purpose of diminishing or decreasing the risk of developing the cancer further. Thus, a prophylactic treatment functions as a preventative treatment against a cancer. In particular embodiments, prophylactic treatments reduce, delay, or prevent metastasis from a primary a cancer tumor site from occurring.

A "therapeutic treatment" includes a treatment administered to a subject who displays symptoms or signs of a cancer and is administered to the subject for the purpose of diminishing or eliminating those signs or symptoms of the cancer. The therapeutic treatment can reduce, control, or eliminate the presence or activity of the cancer and/or reduce control or eliminate side effects of the cancer.

Function as an effective amount, prophylactic treatment or therapeutic treatment are not mutually exclusive, and in particular embodiments, administered dosages may accomplish more than one treatment type.

In particular embodiments, therapeutically effective amounts provide anti-cancer effects. Anti-cancer effects include a decrease in the number of cancer cells, decrease in the number of metastases, a decrease in tumor volume, an increase in life expectancy, induced chemo- or radiosensitivity in cancer cells, inhibited angiogenesis near cancer cells, inhibited cancer cell proliferation, inhibited tumor growth, prevented or reduced metastases, prolonged subject life, reduced cancer-associated pain, and/or reduced relapse or re-occurrence of cancer following treatment.

A "tumor" is a swelling or lesion formed by an abnormal growth of cells (called neoplastic cells or tumor cells). A "tumor cell" is an abnormal cell that grows by a rapid, uncontrolled cellular proliferation and continues to grow after the stimuli that initiated the new growth cease. Tumors show partial or complete lack of structural organization and functional coordination with the normal tissue, and usually form a distinct mass of tissue, which may be benign, pre-malignant or malignant.

For administration, therapeutically effective amounts (also referred to herein as doses) can be initially estimated based on results from in vitro assays and/or animal model studies. Such information can be used to more accurately determine useful doses in subjects of interest. The actual dose amount administered to a particular subject can be determined by a physician, veterinarian or researcher taking into account parameters such as physical and physiological factors including target, body weight, severity of condition, type of cancer, stage of cancer, previous or concurrent therapeutic interventions, idiopathy of the subject and route of administration.

Useful doses can range from 0.1 to 5 µg/kg or from 0.5 to 1 µg /kg. In other non-limiting examples, a dose can include 1 µg /kg, 15 µg /kg, 30 µg /kg, 50 µg/kg, 55 µg/kg, 70 µg/kg, 90 µg/kg, 150 µg/kg, 350 µg/kg, 500 µg/kg, 750 µg/kg, 1000 µg/kg, 0.1 to 5 mg/kg or from 0.5 to 1 mg/kg. In other non-limiting examples, a dose can include 1 mg/kg, 10 mg/kg, 30 mg/kg, 50 mg/kg, 70 mg/kg, 100 mg/kg, 300 mg/kg, 500 mg/kg, 700 mg/kg, 1000 mg/kg or more.

Therapeutically effective amounts can be achieved by administering single or multiple doses during the course of a treatment regimen (e.g., daily, every other day, every 3 days, every 4 days, every 5 days, every 6 days, weekly, every 2 weeks, every 3 weeks, monthly, every 2 months, every 3 months, every 4 months, every 5 months, every 6 months, every 7 months, every 8 months, every 9 months, every 10 months, every 11 months or yearly).

In particular embodiments, BS-BDC can be administered through a pump such as a programmable pump (e.g., an insulin pump). In particular embodiments, staged administration of different BS-BDC can be achieved using, for example, a programmed pump.

In particular embodments, BS-BDC have a short half-life (e.g., short in vivo half-life) such that the BS-BDC are administered using continuous infusion with a pump. In particular embodiments, any BS-BDC with an in vivo half-life of less than 5 hours can be administered through continuous infusion. In contrast, antibodies can have in vivo half-lives of several weeks due to their larger size and Fc portion, and bi-specific formats that contain an Fc portion can similarly have extended in vivo half-lives.

In particular embodiments, therapeutically effective amounts are administered at a time interval to reduce or eliminate cancer recurrence without causing autoimmune toxicity.

The pharmaceutical compositions described herein can be administered by, without limitation, injection, inhalation, infusion, perfusion, lavage or ingestion. Routes of administration can include intravenous, intradermal, intraarterial, intraparenteral, intranasal, intranodal, intralymphatic, intraperitoneal, intralesional, intraprostatic, intravaginal, intrarectal, topical, intrathecal, intratumoral, intramuscular, intravesicular, oral, subcutaneous, and/or sublingual administration and more particularly by intravenous, intradermal, intraarterial, intraparenteral, intranasal, intranodal, intralymphatic, intraperitoneal, intralesional, intraprostatic, intravaginal, intrarectal, topical, intrathecal, intratumoral, intramuscular, intravesicular, oral, subcutaneous, and/or sublingual injection.

As indicated, in particular embodiments, the administration of BS-BDC evolve over time during the course of a subject's treatment regimen. Groups of BS-BDC can combinatorically address many different types of cancer and be customized for individual subjects (e.g., A + B; A + C; A + F; B + F; etc). Likewise, there can be a very personalized aspect to the administration of BS-BDC groups in which subject samples (e.g., liquid biopsies, standard biopsies) are assessed using, for example, polymerase chain reaction (PCR), deep sequencing, flow cytometry, or immunohistochemistry (IHC) to identify emerging clones and to choose pairs of BS-BDC to specifically address an emerging clone. This "cassette" approach can involve monitoring the emergence of resistant clones and rapidly addressing them through new combinations of BS-BDC. "Emerging clone" can refer to a cancer cell or a clonal population of cancer cells with one or more alleles that are distinct from the dominant genotype of the population of cancer cells the clone was derived from. "Drug resistant clone" can refer to a cancer cell or a clonal population of cancer cells that have acquired a new allele that confers resistance to one or more cancer drugs. A patient's cancer can be monitored for the emergence of new cancer clones and/or treatment resistant clones, for example, by sequencing the DNA from a cancer sample derived from the patient.

In particular embodiments, a patient can be monitored for immune suppression in the tumor microenvironment and/or T-cell suppression. Immune suppression in the microenvironment and/or T-cell suppression can be monitored, for example, by measuring cytokine levels and/or the number of T-cells in a sample derived from the patient.

Methods disclosed herein include activating immune cells in the tumor microenvironment. In particular embodiments, activating immune cells in the tumor microenvironment includes reducing or reversing T cell suppression in the tumor microenvironment. T cell suppression can refer to a block of or reduction in T cell activation, such as can be caused by regulatory T cells. Methods to measure T cell suppression can be found, for example in McMurchy & Levings, European Journal of Immunology 42(1): 27-34. Reducing or reversing T cell suppression in the tumor microenvironment can include replacing a CD28-binding BS-BDC with a BS-BDC that reduces the activity of an immune cell suppressor. This approach is beneficial when T cells in the tumor microenvironment reduce expression of CD28 following on-going activation.

Example 1. The Fred Hutchinson Cancer Research Center (FHCRC) Antibody Development Facility and the Molecular Design Therapeutics Core will be used to enable a rational, computational protein design approach for the development and humanization of novel "clinic ready" SMITE antibody therapeutics. A description of the human material used in this research is provided below.

Healthy donor T-cells: Unstimulated mononuclear cells will be collected from healthy adult volunteers via leukapheresis by the FHCRC Hematopoietic Cell Processing Core under IRB-approved research protocols as used in prior bispecific antibody studies. T-cells will be enriched through magnetic cell sorting, and then frozen in de-identified fashion in aliquots and stored in liquid nitrogen until use. Thawed cell aliquots will be labeled with CellBue Burgundy to allow separation from cancer cells.

T-cell co-stimulation is required for maximum activity of bispecific T-cell engaging antibodies: Acute leukemia cell lines and genetically engineered sublines were used to test the impact of inhibitory (PD-L1 and PD-L2) and activating (CD80 and CD86) T-cell ligands on the in vitro activity of the CD33/CD3 and CD19/CD3 antibodies, AMG330 and blinatubmomab. Next these experiments were repeated using specimens obtained from acute leukemia patients. The results demonstrated that expression of PD-L1 or PD-L2 reduced the cytolytic activity of bispecific T-cell engaging antibodies, whereas expression of CD80 or CD86 augmented their activity. Consistent with this, co-treatment with an activating antibody directed at the co-stimulatory T-cell receptor, CD28, significantly increased bispecific T-cell engaging antibody-induced cytotoxicity in acute leukemia cell lines. In 12 AML patient specimens, simultaneous activation of CD28 also increased the activity of AMG330 in primary leukemia cells (P=0.023). Together, these findings indicate that T-cell co-receptor activation is required for maximum activity of bispecific T-cell engaging antibodies and suggest that provision of a co-stimulatory signal to T-cells can overcome resistance to these agents. Previous studies from other investigators have indicated that CD3 x CD28 cross-reactive bispecific antibodies may provide a large therapeutic window where only tumor cell dependent T-cell activation is induced and systemic tumor cell independent T-cell activation is avoided.

In particular embodiments, the SMITE antibody approach requires the concomitant use of two bispecific T-cell engaging antibodies, one directed at CD3 (or CD8) and the other directed at CD28. To relay a maximal activation signal to T-cells, both antibodies need to bind ROR1 in a time-overlapped fashion. For these studies, well validated, publicly available sequences from three ROR1 antibodies can be used. In the initial antibody set of interest, the ROR1-A antibody (clone: R11) and ROR1-a antibody (clone: 2A2) bind the same epitope and compete with each other for binding to ROR1. The ROR1-B antibody (clone: R12) binds a non-overlapping proximal epitope and can bind ROR1 simultaneously with either one of the other two antibodies. Using the scFvs of these three ROR1 antibodies, as well as scFvs of publicly available antibodies recognizing CD3 (clone: OKT3), CD8 (clone: OKT8) and CD28 (clone: 9D7), bispecific T-cell engaging antibodies will be generated as building blocks with swappable binding modules to form a flexible ROR1-directed SMITE antibody platform (see, e.g., Table 1). All antibodies will be generated as "hisavi" constructs including the 6xhistidine tag for purification and an avitag for specific biotinylation by Bir-A ligase.

Single-chain constructs targeting ROR1 from the ROR1-A, ROR1-a, and ROR1-B antibodies have been designed as expressed. As evidenced by surface plasmon resonance (SPR) analyses using Biacore chips, these single-chain constructs retain robust affinity for the ROR1 antigen. Using size-exclusion chromatography, single-chain constructs derived from ROR1-A and ROR1-B were then demonstrated to bind simultaneously to ROR1.Subsequently, two bispecific T-cell engaging antibody molecules were designed and successfully expressed. Using 2-liter preps, yields of 22.4mg and 14.4mg were obtained for the aROR1-a/aCD28 and the aROR1-B/aCD3 construct, respectively. In both productions, little aggregation, degradation, or misfolding was observed and the aROR1-B/aCD3 construct was confirmed to bind ROR1, demonstrating successful conversion of antibody sequences into high-quality bispecific T-cell engaging antibodies.

Experimental approach: Antibody generation: All antibodies will be generated using the customized Daedalus lentiviral transduction system as described previously. Briefly, each construct will be cloned with a cleavable C-terminal 6xHis-Avi tag into a parental expression plasmid (including an IRES-GFP) and co-transfected (along with psPAX2 and pMD2G) into 293T-cells stably expressing the BirA biotin ligase using polyethylenimine (PEI). The resulting lentivirus will be used to transduce suspension-adapted 293F cells, and protein expression will be monitored using GFP. Secreted antibodies will be harvested 2 weeks after transduction and then purified from conditioned media using conventional affinity chromatography. Size-exclusion chromatography and SDS-PAGE will then be used to determine the stability and aggregation tendency of individual molecules.

Binding of individual bispecific T-cell engaging antibodies to ROR1 captured on SPR biosensors will be quantified on a Biacore T100 instrument (GE Healthcare) as previously described (see, e.g., Finton et al., Autoreactivity and exceptional CDR plasticity (but not unusual polyspecificity) hinder elicitation of the anti-HIV antibody 4E10. PLoS Pathog. 2013;9(9):e1003639 and Finton et al., Ontogeny of recognition specificity and functionality for the broadly neutralizing anti-HIV antibody 4E10. PLoS Pathog. 2014; 10(9):e1004403) and successfully performed on early ROR1 scFvs as summarized above. As reagents are currently not available to allow SPR interaction analyses between bispecific T-cell engaging antibodies and CD3, CD8, or CD28, appropriate target antigen positive/negative cell lines, together with anti-His antibodies, will be used to measure binding of antibody molecules to T-cells using flow cytometry-based assays.

Cytolytic properties of all bispecific T-cell engaging antibodies will be determined in comparative in vitro assays that have been successfully employed to characterize other bispecific T-cell engaging antibodies. ROR1+ primary tumor cells (JeKo) and transfected cells (K562/ROR1) are available for these studies, and ROR1 expression constructs that permit the generation of additional cell lines if necessary are also available. Appropriate ROR1- cells (e.g. parental K562 cells or MKN45 cells) will serve as negative controls. ROR1+ or ROR1- cell lines will be incubated in 96-well round bottom plates at 5-10,000 cells/well in 225 µL of appropriate culture medium including various concentrations of individual bispecific T-cell engaging antibodies in the presence or absence of healthy donor T-cells added at different E:T-cell ratios. After 48 hours, cell numbers and drug-induced cytotoxicity, using 4',6-diamidino-2-phenylindole (DAPI) to detect non-viable cells, will be determined using a LSRII cytometer. In experiments where healthy donor T-cells are added, cancer cells will be identified by forward/side scatter properties and negativity for CellVue Burgundy dye.

The generated BS-BDC group, in particular those directed at CD3, will have potent cytolytic properties when used alone. Based on the preliminary findings, however, it is anticipated that the efficacy of bispecific T-cell engaging antibodies can be augmented if they are used in groups such that they also activate T- cell co-stimulatory signaling. It is expected that a combination of CD3- and CD28-directed antibodies will provide the best response, but this modular system allows for determining empirically the best combination of antibodies without limiting the search to an a priori determined set of molecules. These bispecific T-cell engaging antibody groups will also be useful to show that simultaneous targeting of two non-overlapping ROR1 epitopes (while targeting one or two T-cell antigens) provides an advantage over targeting a single ROR1 epitope. Antibody groups with the most favorable biophysical and cytolytic properties will be identified. Next, the selected antibody groups will be humanized. Most antibodies in the clinic today are humanized versions of mouse antibodies. Although the murine forms of the bispecific T-cell engaging molecules could have utility, immunogenicity can be a liability in clinical development. As humanization is an accepted technology to minimize the formation of neutralizing antibodies, this step is considered essential for their clinical development, and thus will be incorporated into the earliest possible stage of candidate molecule development.

Experimental approach: Groups of bispecific T-cell engaging antibodies were rationally selected based on specificities for T-cell antigens (e.g. CD3 and CD28 or CD8 and CD28) and non-overlapping ROR1 epitope recognition (e.g. ROR1- A and ROR1-B or ROR1-a and ROR1-B). Besides these groups of potential interest, a few groups that are predicted to be less suited as SMITE antibodies (e.g. ROR1-A and ROR1-a, or CD3 and CD8) will also be tested. Next, these antibody groups will be subjected to analyses of target antigen binding and determination of drug-induced cytotoxicity. Individual antibodies used alone will serve for comparative analyses. In addition, the ability of SMITE antibody constructs to elicit T-cell cytokine release in the presence of ROR1-expressing cells will be assessed in co-culture experiments by ELISA and intracellular flow cytometry. Antibody groups will then be ranked based on biophysical and cytolytic properties, and groups of highest interest will be subjected to antibody humanization, using labor-intensive standard methodologies routinely available in the FHCRC core facility. This approach will primarily be based on Complementary Determining Region (CDR) grafting with mutation of vernier zone residues back to murine as needed to retain binding, with a focus on surface residues, if it is determined that a large number of murine vernier zone residues are needed. The Molecular Therapeutic Core has a 24-well, robotic transduction and expression system well-suited for the generation of the large numbers of candidate molecules.

Groups of BS-BDC that have better cytolytic properties together than when used individually will be identified. However, it is conceivable that the simultaneous engagement of two bispecific T-cell engaging antibodies requires adjustment of the linker length of at least one of the BS-BDC. Use of a robotic expression facility will allow for several iterations of linker design if necessary.

Groups of humanized ROR1-directed antibodies will be generated to identify lead candidate molecules that can then be tested in more extensive preclinical studies (e.g. for toxicity properties in large animals) and, ultimately, be brought to the clinic.

Constructs of interest will be tested for their ability to mediate anti-tumor activity in NSG mice engrafted with human T- cells and firefly luciferase-expressing ROR1+ tumor cells (JeKo and MDA-MB231) that represent hematological (JeKo) and solid tumor (MDA-MB231) cell models. NSG mice will also be used for studies to determine serum half-lives of antibodies of interest. In these studies, blood will be collected by cardiac puncture at euthanasia and analyzed by mass spectrometry or scintillation counting. These studies will identify lead candidate humanized antibody bispecific T-cell engaging molecule(s) that can be used for further testing.

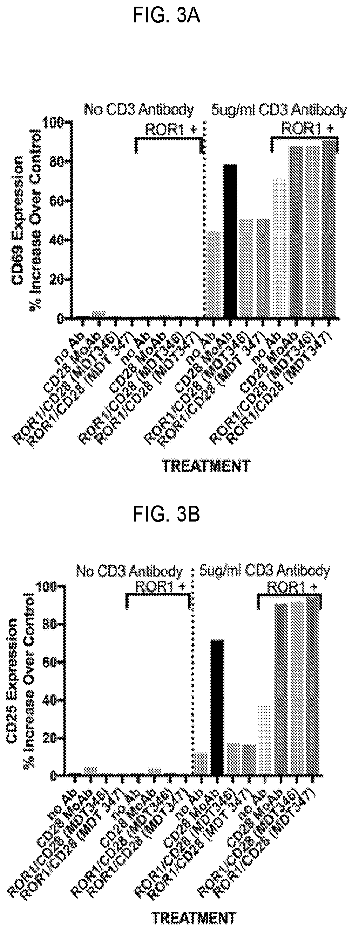

Example 2. FIGs. 3A and 3B show that T-cell co-activation with CD28-directed bispecific antibodies is strictly dependent on presence of target antigen-positive cancer cells. In these experiments, ROR1-negative parental K562 cells or K562 cells expressing ROR1 were incubated in wells coated with CD3 antibody (clone OKT3) together with healthy donor T-cells at an E:T ratio of 1:1 with or without a monoclonal CD28 antibody or a ROR1/CD28 antibody as indicated. After 48 hours, T-cell activation was quantified by flow cytometry via determination of cell surface expression of CD69 and CD25. T- cell activation was almost exclusively seen when an activating CD3 antibody was present. In the presence of ROR1-negative cancer cells, T-cell co-activation was possible with an activating CD28 monoclonal antibody but not with ROR1/CD28 antibodies. On the other hand, in the presence of ROR1-positive cancer cells, T-cell co-activation was possible with both a monoclonal CD28 antibody as well as ROR1/CD28 antibodies. Together, these data are consistent with the notion of cancer cell target ("nonspecific") T-cell activation when monoclonal CD28 antibodies were used, whereas T-cell co-activation with CD28-directed bispecific T-cell engaging antibodies was as efficient as that seen with a monoclonal CD28 antibody but strictly depended on the presence of target antigen-positive cancer cells.

FIGs. 4A-4D show that T-cell co-activation with CD28-directed bispecific antibody augments ROR1/CD3 antibody-induced cytotoxicity. K562 cells forced to express ROR1 (K562/ROR1) were incubated with healthy donor T-cells at an E:T ratio of 1:1 in the presence of the ROR1/CD3 antibody MDT319 (including variable domains from the ROR1 antibody, clone 2A2) (4A, 4B) or the ROR1/CD3 antibody MDT320 (including variable domains from the non-cross-reactive ROR1 antibody, clone R12) (4C, 4D) with or without various concentrations of either the ROR1/CD28 antibody MDT347 (including variable domains from the ROR1 antibody, clone R12) (4A, 4C) or a monoclonal CD28 antibody (clone CD28.2) (4B, 4D) as indicated. After 48 hours, cell numbers and drug-induced cytotoxicity were determined. Both ROR1/CD3 antibodies cause dose-dependent cytotoxicity in ROR1-transduced K562 cells. This cytotoxicity can be significantly augmented via co-treatment with either a monoclonal CD28 antibody or a ROR1/CD28 antibody. The magnitude of this augmenting effect is comparable between the monoclonal CD28 antibody and the ROR1/CD28 antibody.

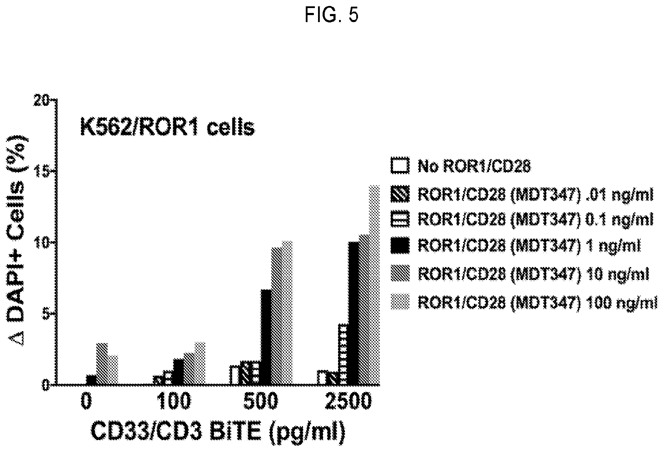

FIG. 5 shows that T-cell co-activation with CD28-directed bispecific antibodies targeting a second cancer cell antigen can augment anti-cancer activity of therapeutic bispecific T-cell engaging antibody. CD33^{dim+} K562 cells forced to express ROR1 (K562/ROR1) were incubated with healthy donor T-cells at an E:T ratio of 1:1 in the presence of a CD33/CD3 antibody with or without various concentrations of a ROR1/CD28 antibody (MDT347) as indicated. After 48 hours, cell numbers and drug-induced cytotoxicity were determined. In these K562 cells that only express low levels of CD33, the CD33/CD3 antibody has limited single agent activity. Combination with a second antibody that targets ROR1 and co-activates CD28 synergizes in a dose-dependent fashion with the CD33/CD3 antibody and substantially increases drug-induced cytotoxicity.

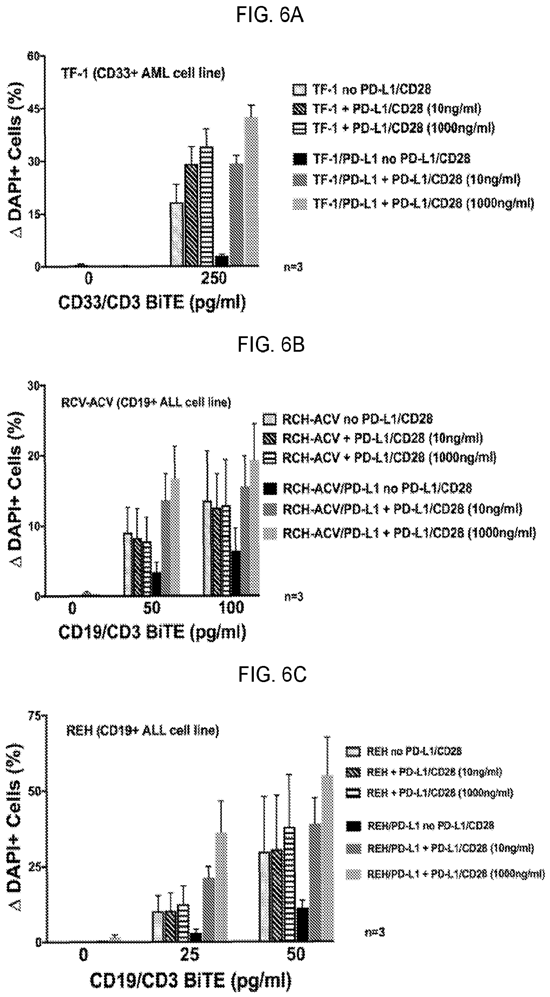

FIGs. 6A-6C show that PD-L1/CD28 antibody can overcome PD-L1-mediated resistance to bispecific antibodies. Parental CD33+ TF-1 cells (6A), CD19+ RCV-ACV cells (6B), or CD19+ REH cells (6C) and corresponding sublines over-expressing PD-L1 were incubated with healthy donor T-cells at an E:T ratio of 1:1 in the presence of a CD33/CD3 or CD19/CD3 antibody as appropriate with or without a PD-L1/CD28 antibody (MDT359) as indicated. After 48 hours, cell numbers and drug-induced cytotoxicity were determined. As demonstrated previously (Laszlo et al, Blood Cancer J 2015), over-expression of PD-L1 leads to relative of resistance of leukemia cells to bispecific antibody-induced cytotoxicity. A PD-L1/CD28 antibody is able to fully overcome this resistance.

Once lead candidate antibodies have been identified, larger-scale production of clinical-grade antibodies for preclinical safety and initial human clinical trials will be initiated. It is important to note that the Biologics Production Facility at FHCRC, as a current Good Manufacturing Processes (cGMP) laboratory, can generate validated biologics for Phase 1/2 studies. For example, the scFv to CD3, derived from the OKT3 antibody, is the same as the one utilized in blinatumomab, whereas the CD28 antibody that is being derived from scFv sequences has been demonstrated to activate T-cells in vivo.

Statistical considerations: Predominantly, standard descriptive statistics for paired analyses will be used, which will be performed in consultation with a biostatistician.

As will be understood by one of ordinary skill in the art, each embodiment disclosed herein can comprise, consist essentially of or consist of its particular stated element, step, ingredient or component. Thus, the terms "include" or "including" should be interpreted to recite: "comprise, consist of, or consist essentially of." The transition term "comprise" or "comprises" means includes, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the embodiment to the specified elements, steps, ingredients or components and to those that do not materially affect the embodiment. A material effect would cause a statistically-significant reduction in T cell activation following binding of a BS-BDC group to a cancer cell.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Definitions and explanations used in the present disclosure are meant and intended to be controlling in any future construction unless clearly and unambiguously modified in the following examples or when application of the meaning renders any construction meaningless or essentially meaningless. In cases where the construction of the term would render it meaningless or essentially meaningless, the definition should be taken from Webster's Dictionary, 3rd Edition or a dictionary known to those of ordinary skill in the art, such as the Oxford Dictionary of Biochemistry and Molecular Biology (Ed. Anthony Smith, Oxford University Press, Oxford, 2004).

## Claims

1. A group of at least two bi-specific binding domain constructs (BS-BDC) wherein each BS-BDC in the group targets
(i) a cancer antigen epitope that is non-overlapping and non-repetitive with a cancer antigen epitope targeted by another BS-BDC within the group and
(ii) an immune cell activating epitope that is non-overlapping and non-repetitive with an immune cell activating epitope targeted by another BS-BDC within the group,
wherein two of the non-overlapping and non-repetitive cancer antigen epitopes are located on ROR1 and one of the non-overlapping and non-repetitive immune cell activating epitopes is located on CD3 and one of the non-overlapping and non-repetitive immune cell activating epitopes is located on CD28,
wherein, at least one BS-BDC comprises a scFV comprising a CDRL1 (SEQ ID NO: 18), CDRL2 (SEQ ID NO: 19), CDRL3 (SEQ ID NO: 20), CDRH1 (SEQ ID NO: 21), CDRH2 (SEQ ID NO: 22), and CDRH3 sequence (SEQ ID NO: 23) of the R11 antibody,
or wherein at least one BS-BDC comprises a scFV comprising a CDRL1 (SEQ ID NO: 24), CDRL2 (SEQ ID NO: 25), CDRL3 (SEQ ID NO: 26), CDRH1 (SEQ ID NO: 27), CDRH2 (SEQ ID NO: 28), and CDRH3 (SEQ ID NO: 29) sequence of the R12 antibody,
or wherein at least one BS-BDC comprises a scFV comprising a CDRL1 (SEQ ID NO: 30) CDRL2 (SEQ ID NO: 31), CDRL3 (SEQ ID NO: 32), CDRH1 (SEQ ID NO: 33), CDRH2 (SEQ ID NO: 34), and CDRH3 (SEQ ID NO: 35) sequence of the 2A2 antibody,
or wherein at least one BS-BDC comprises a scFV comprising a CDRL1 (SEQ ID NO: 100), CDRL2 (SEQ ID NO: 101), CDRL3 (SEQ ID NO: 102), CDRH1 (SEQ ID NO: 103), CDRH2 (SEQ ID NO: 104), and CDRH3 (SEQ ID NO: 105) sequence of the OKT3 antibody,
or wherein at least one BS-BDC comprises a scFV comprising a CDRL1 (SEQ ID NO: 131), CDRL2 (SEQ ID NO: 132), CDRL3 (SEQ ID NO: 133), CDRH1 (SEQ ID NO: 134), CDRH2 (SEQ ID NO: 135), and CDRH3 (SEQ ID NO: 136) sequence of the TGN1412 antibody,
or comprising SEQ ID NO: 238,
or comprising SEQ ID NO: 239,
or comprising SEQ ID NO: 240,
or comprising SEQ ID NO: 241,
or comprising SEQ ID NO: 242,
or comprising SEQ ID NO: 243,
or comprising SEQ ID NO: 244,
or comprising SEQ ID NO: 245.

2. The group of claim 1, comprising at least two BS-BDC selected from SEQ ID NO: 238; SEQ ID NO: 239; SEQ ID NO: 240; SEQ ID NO: 241; SEQ ID NO: 242; SEQ ID NO: 243; SEQ ID NO: 244; SEQ ID NO: 245; and SEQ ID NO: 247.

3. The group of claim 1, comprising three or four BS-BDC selected from SEQ ID NO: 238; SEQ ID NO: 239; SEQ ID NO: 240; SEQ ID NO: 241; SEQ ID NO: 242; SEQ ID NO: 243; SEQ ID NO: 244; SEQ ID NO: 245; and SEQ ID NO: 247.

4. The group of any one of claims 1-3 wherein the BS-BDCs are scFvs.

5. A composition comprising the group of any one of claims 1-4.

6. The composition of claim 5 for use in treating cancer in a subject in need thereof comprising administering a therapeutically amount of the composition to a subject in need thereof.

7. The composition for use of claim 6 wherein the treating overcomes resistance of a cancer cell to a treatment.

8. The composition for use of claim 6 or claim 7, further comprising monitoring the subject for changes in the subject's cancer.

9. The composition for use of claim 8, further comprising administering another composition with a different BS-BDC group including at least two BS-BDCs from the BS-BDCs of any one of claims 2, 3 and 4, based at least in part on the monitoring.

10. The composition for use of claim 8 or claim 9, further comprising administering at least two BS-BDC selected from SEQ ID NO: 246; SEQ ID NO: 248; SEQ ID NO: 249; SEQ ID NO: 250; SEQ ID NO: 251 and SEQ ID NO: 252, based at least in part on the monitoring.

11. The composition for use of claim 8, wherein results of the monitoring indicate emergence of a clone.

12. The composition for use of claim 8, wherein results of the monitoring indicate immune suppression in a tumor microenvironment corresponding to the cancer cell.

13. The composition for use of claim 8, wherein results of the monitoring indicate T cell suppression in a tumor microenvironment corresponding to the cancer cell.

14. The composition of claim 5 for use in stimulating an immune response in a subject in need thereof comprising administering a therapeutically amount of the composition to a subject in need thereof.

## Patentansprüche

1. Gruppe von mindestens zwei bispezifischen Bindungsdomänenkonstrukten (BS-BDC), wobei jedes BS-BDC in der Gruppe abzielt auf
(i) ein Krebsantigenepitop, das mit einem Krebsantigenepitop, auf das ein anderes BS-BDC innerhalb der Gruppe abzielt, nicht überlappend und nicht repetitiv ist, und
(ii) ein immunzellaktivierendes Epitop, das mit einem immunzellaktivierenden Epitop, auf das ein anderes BS-BDC innerhalb der Gruppe abzielt, nicht überlappend und nicht repetitiv ist,
wobei zwei der nicht überlappenden und nicht repetitiven Krebsantigenepitope auf ROR1 lokalisiert sind und eines der nicht überlappenden und nicht repetitiven immunzellaktivierenden Epitope auf CD3 lokalisiert ist und eines der nicht überlappenden und nicht repetitiven immunzellaktivierenden Epitope auf CD28 lokalisiert ist,
wobei mindestens ein BS-BDC ein scFV umfasst, umfassend eine CDRL1- (SEQ ID NO: 18), CDRL2- (SEQ ID NO: 19), CDRL3- (SEQ ID NO: 20), CDRH1- (SEQ ID NO: 21), CDRH2- (SEQ ID NO: 22) und CDRH3-Sequenz (SEQ ID NO: 23) des R11-Antikörpers,
oder wobei mindestens ein BS-BDC ein scFV umfasst, umfassend eine CDRL1- (SEQ ID NO: 24), CDRL2- (SEQ ID NO: 25), CDRL3- (SEQ ID NO: 26), CDRH1- (SEQ ID NO: 27), CDRH2- (SEQ ID NO: 28) und CDRH3-(SEQ ID NO: 29)Sequenz des R12-Antikörpers,
oder wobei mindestens ein BS-BDC ein scFV umfasst, umfassend eine CDRL1- (SEQ ID NO: 30) CDRL2- (SEQ ID NO: 31), CDRL3- (SEQ ID NO: 32), CDRH1- (SEQ ID NO: 33), CDRH2- (SEQ ID NO: 34) und CDRH3-(SEQ ID NO: 35)Sequenz des 2A2-Antikörpers,
oder wobei mindestens ein BS-BDC ein scFV umfasst, umfassend eine CDRL1- (SEQ ID NO: 100), CDRL2- (SEQ ID NO: 101), CDRL3-(SEQ ID NO: 102), CDRH1- (SEQ ID NO: 103), CDRH2- (SEQ ID NO: 104) und CDRH3-(SEQ ID NO: 105)Sequenz des OKT3-Antikörpers,
oder wobei mindestens ein BS-BDC ein scFV umfasst, umfassend eine CDRL1- (SEQ ID NO: 131), CDRL2- (SEQ ID NO: 132), CDRL3-(SEQ ID NO: 133), CDRH1- (SEQ ID NO: 134), CDRH2- (SEQ ID NO: 135) und CDRH3-(SEQ ID NO: 136)Sequenz des TGN1412-Antikörpers,
oder umfassend SEQ ID NO: 238,
oder umfassend SEQ ID NO: 239,
oder umfassend SEQ ID NO: 240,
oder umfassend SEQ ID NO: 241,
oder umfassend SEQ ID NO: 242,
oder umfassend SEQ ID NO: 243,
oder umfassend SEQ ID NO: 244,
oder umfassend SEQ ID NO: 245.

2. Gruppe nach Anspruch 1, umfassend mindestens zwei BS-BDC, die aus SEQ ID NO: 238; SEQ ID NO: 239; SEQ ID NO: 240; SEQ ID NO: 241; SEQ ID NO: 242; SEQ ID NO: 243; SEQ ID NO: 244; SEQ ID NO: 245; und SEQ ID NO: 247 ausgewählt sind.

3. Gruppe nach Anspruch 1, umfassend drei oder vier BS-BDC, die aus SEQ ID NO: 238; SEQ ID NO: 239; SEQ ID NO: 240; SEQ ID NO: 241; SEQ ID NO: 242; SEQ ID NO: 243; SEQ ID NO: 244; SEQ ID NO: 245; und SEQ ID NO: 247 ausgewählt sind.

4. Gruppe nach einem der Ansprüche 1 bis 3, wobei die BS-BDCs scFvs sind.

5. Zusammensetzung, umfassend die Gruppe nach einem der Ansprüche 1 bis 4.

6. Zusammensetzung nach Anspruch 5 zur Verwendung bei einem Behandeln von Krebs bei einem Subjekt, das dessen bedarf, umfassend ein Verabreichen einer therapeutischen Menge der Zusammensetzung an ein Subjekt, das dessen bedarf.

7. Zusammensetzung zur Verwendung nach Anspruch 6, wobei das Behandeln eine Resistenz einer Krebszelle gegen eine Behandlung überwindet.

8. Zusammensetzung zur Verwendung nach Anspruch 6 oder 7, ferner umfassend ein Überwachen des Subjekts auf Veränderungen der Krebserkrankung des Subjekts.

9. Zusammensetzung zur Verwendung nach Anspruch 8, ferner umfassend das Verabreichen einer anderen Zusammensetzung mit einer unterschiedlichen BS-BDC-Gruppe, die mindestens zwei BS-BDCs aus den BS-BDCs nach einem der Ansprüche 2, 3 und 4 einschließt, basierend mindestens teilweise auf dem Überwachen.

10. Zusammensetzung zur Verwendung nach Anspruch 8 oder 9, ferner umfassend das Verabreichen von mindestens zwei BS-BDC, die aus SEQ ID NO: 246; SEQ ID NO: 248; SEQ ID NO: 249; SEQ ID NO: 250; SEQ ID NO: 251 und SEQ ID NO: 252 ausgewählt sind, basierend mindestens teilweise auf dem Überwachen.

11. Zusammensetzung zur Verwendung nach Anspruch 8, wobei Ergebnisse des Überwachens ein Auftreten eines Klons anzeigen.

12. Zusammensetzung zur Verwendung nach Anspruch 8, wobei Ergebnisse des Überwachens eine Immunsuppression in einer Tumormikroumgebung anzeigen, die der Krebszelle entspricht.

13. Zusammensetzung zur Verwendung nach Anspruch 8, wobei Ergebnisse des Überwachens eine T-Zell-Suppression in einer Tumormikroumgebung anzeigen, die der Krebszelle entspricht.

14. Zusammensetzung nach Anspruch 5 zur Verwendung bei einem Stimulieren einer Immunantwort bei einem Subjekt, das dessen bedarf, umfassend das Verabreichen einer therapeutischen Menge der Zusammensetzung an ein Subjekt, das dessen bedarf.

## Revendications

1. Groupe d'au moins deux structures de domaines de liaison bispécifiques (BS-BDC), dans lequel chaque BS-BDC dans le groupe cible
(i) un épitope antigénique du cancer qui est sans chevauchement et non répétitif avec un épitope antigénique du cancer ciblé par une autre BS-BDC au sein du groupe et
(ii) un épitope d'activation de cellules immunitaires qui est sans chevauchement et non répétitif avec un épitope d'activation de cellules immunitaires ciblé par une autre BS-BDC au sein du groupe,
dans lequel deux des épitopes antigéniques du cancer sans chevauchement et non répétitifs sont situés sur ROR1 et l'un des épitopes d'activation de cellules immunitaires sans chevauchement et non répétitifs est situé sur CD3 et l'un des épitopes d'activation de cellules immunitaires sans chevauchement et non répétitifs est situé sur CD28,
dans lequel, au moins une BS-BDC comprend un scFv comprenant une séquence CDRL1 (SEQ ID NO : 18), CDRL2 (SEQ ID NO : 19), CDRL3 (SEQ ID NO : 20), CDRH1 (SEQ ID NO : 21), CDRH2 (SEQ ID NO : 22) et CDRH3 (SEQ ID NO : 23) de l'anticorps R11,
ou dans lequel au moins une BS-BDC comprend un scFv comprenant une séquence CDRL1 (SEQ ID NO : 24), CDRL2 (SEQ ID NO : 25), CDRL3 (SEQ ID NO : 26), CDRH1 (SEQ ID NO : 27), CDRH2 (SEQ ID NO : 28) et CDRH3 (SEQ ID NO : 29) de l'anticorps R12,
ou dans lequel au moins une BS-BDC comprend un scFv comprenant une séquence CDRL1 (SEQ ID NO : 30), CDRL2 (SEQ ID NO : 31), CDRL3 (SEQ ID NO : 32), CDRH1 (SEQ ID NO : 33), CDRH2 (SEQ ID NO : 34) et CDRH3 (SEQ ID NO : 35) de l'anticorps 2A2,
ou dans lequel au moins une BS-BDC comprend un scFv comprenant une séquence CDRL1 (SEQ ID NO : 100), CDRL2 (SEQ ID NO : 101), CDRL3 (SEQ ID NO : 102), CDRH1 (SEQ ID NO : 103), CDRH2 (SEQ ID NO : 104) et CDRH3 (SEQ ID NO : 105) de l'anticorps OKT3,
ou dans lequel au moins une BS-BDC comprend un scFv comprenant une séquence CDRL1 (SEQ ID NO : 131), CDRL2 (SEQ ID NO : 132), CDRL3 (SEQ ID NO : 133), CDRH1 (SEQ ID NO : 134), CDRH2 (SEQ ID NO : 135) et CDRH3 (SEQ ID NO : 136) de l'anticorps TGN1412,
ou comprenant SEQ ID NO : 238,
ou comprenant SEQ ID NO : 239,
ou comprenant SEQ ID NO : 240,
ou comprenant SEQ ID NO : 241,
ou comprenant SEQ ID NO : 242,
ou comprenant SEQ ID NO : 243,
ou comprenant SEQ ID NO : 244,
ou comprenant SEQ ID NO : 245.

2. Groupe selon la revendication 1, comprenant au moins deux BS-BDC choisies parmi SEQ ID NO : 238 ; SEQ ID NO : 239 ; SEQ ID NO : 240 ; SEQ ID NO : 241 ; SEQ ID NO : 242 ; SEQ ID NO : 243 ; SEQ ID NO : 244 ; SEQ ID NO : 245 ; et SEQ ID NO : 247.

3. Groupe selon la revendication 1, comprenant trois ou quatre BS-BDC choisies parmi SEQ ID NO : 238 ; SEQ ID NO : 239 ; SEQ ID NO : 240 ; SEQ ID NO : 241 ; SEQ ID NO : 242 ; SEQ ID NO : 243 ; SEQ ID NO : 244 ; SEQ ID NO : 245 ; et SEQ ID NO : 247.

4. Groupe selon l'une quelconque des revendications 1 à 3, dans lequel les BS-BCD sont des scFv.

5. Composition comprenant le groupe selon l'une quelconque des revendications 1 à 4.

6. Composition selon la revendication 5 pour une utilisation dans le traitement du cancer chez un sujet qui en a besoin, comprenant l'administration d'une quantité thérapeutiquement de la composition à un sujet qui en a besoin.

7. Composition pour une utilisation selon la revendication 6, dans laquelle le traitement surmonte la résistance d'une cellule cancéreuse à un traitement.

8. Composition pour une utilisation selon la revendication 6 ou la revendication 7, comprenant en outre la surveillance du sujet pour détecter des changements du cancer du sujet.

9. Composition pour une utilisation selon la revendication 8, comprenant en outre l'administration d'une autre composition avec un groupe de BS-BDC différent comportant au moins deux BS-BDC parmi les BS-BDC selon l'une quelconque des revendications 2, 3 et 4, sur la base au moins en partie de la surveillance.

10. Composition pour une utilisation selon la revendication 8 ou la revendication 9, comprenant en outre l'administration d'au moins deux BS-BDC choisies parmi SEQ ID NO : 246 ; SEQ ID NO : 248 ; SEQ ID NO : 249 ; SEQ ID NO : 250 ; SEQ ID NO : 251 et SEQ ID NO : 252, sur la base au moins en partie de la surveillance.

11. Composition pour une utilisation selon la revendication 8, dans laquelle les résultats de la surveillance indiquent l'émergence d'un clone.

12. Composition pour une utilisation selon la revendication 8, dans laquelle les résultats de la surveillance indiquent une immunosuppression dans un microenvironnement tumoral correspondant à la cellule cancéreuse.

13. Composition pour une utilisation selon la revendication 8, dans laquelle les résultats de la surveillance indiquent une suppression des lymphocytes T dans un microenvironnement tumoral correspondant à la cellule cancéreuse.

14. Composition selon la revendication 5, pour une utilisation dans la stimulation d'une réponse immunitaire chez un sujet qui en a besoin, comprenant l'administration d'une quantité thérapeutiquement de la composition à un sujet qui en a besoin.
